# EUROPEAN PATENT APPLICATION

(11) **EP 4 454 649 A1**
(43) Date of publication of application: **30.10.2024**
(21) Application number: 22911253.7
(22) Date of filing: 21.12.2022
(51) Int. Cl.: A61K 31/485, A61P 25/18

(54) **USE OF OPIOID FOR TREATMENT OF AUTISM SPECTRUM DISORDERS**

(30) Priority: 21.12.2021 JP 2021206572
(71) Applicant: OSAKA UNIVERSITY, Suita-shi Osaka 565-0871 (JP); Shionogi & Co., Ltd, Osaka-shi, Osaka 541-0045 (JP)
(72) Inventor: AGO Yukio, Suita-shi, Osaka 565-0871 (JP); YAMAKAWA Hidekuni, Osaka-shi, Osaka 541-0045 (JP); NAKAMURA Atsushi, Osaka-shi, Osaka 541-0045 (JP)
(74) Representative: Vossius & Partner Patentanwälte Rechtsanwälte mbB
(86) International application number: PCT/JP2022/047004
(87) International publication number: WO 2023/120551

(57) **Abstract**

Provided are a new pharmaceutical composition and a method for treating and/or preventing autism spectrum disorder, fragile X syndrome, and/or an autism spectrum disorder-like symptom. A pharmaceutical composition for the treatment and/or prevention of autism spectrum disorder, fragile X syndrome, and/or an autism spectrum disorder-like symptom, comprising buprenorphine or a pharmaceutically acceptable salt thereof and/or morphine or a pharmaceutically acceptable salt thereof.

## Description

### [TECHNICAL FIELD]

The present invention relates to new pharmaceutical compositions and methods for the treatment and/or prevention of autism spectrum disorder, fragile X syndrome, autism spectrum disorder-like symptoms and/or disorders in social communication and/or social interaction, particularly preferably for the treatment of autism spectrum disorder.

### [BACKGROUND ART]

Autism spectrum disorder (ASD) is a disease characterized by the presence of persistent disorders in social communication and social interaction in multiple contexts, the presence of two or more limited, repetitive patterns of behavior/interest or activity (emotional, repetitive body movements and conversations, persistence and obsessiveness, extremely limited and preoccupied interest, hypersensitivity or obtundation to sensory stimuli, etc.), and the presence of these symptoms from early development. There are 3.8 million patients in the world (Non-patent Document 1) and 350,000 or more patients in Japan, and it is said that the economic burden required per patient is 5 million yen or more per year (Non-patent Document 2), and in many cases, considering that the disease period is a lifetime, the economic influence is very large. However, there is currently no effective treatment agent for the core symptoms of ASD. As a treatment for ASD, behavior therapy such as applied behavior analysis is central, but since behavior therapy requires a lot of time and human resources, and it is known that the effect decreases as the age increases, patients who can enjoy the benefit are very limited. From the above background, there is a strong demand for a treatment agent effective for core symptoms of ASD.

At present, as a target molecule of an ASD treatment agent, for example, a molecule known to be associated with social behavior such as oxytocin or vasopressin and a receptor thereof are attracting attention.

There are several reports of opioid receptors and social behaviors. In 1979, a theory that ASD symptoms are caused by an excessive opioid signal in the brain was proposed (Non-patent Document 3), and thereafter, a clinical trial was conducted on the ASD treatment effect of Naltrexone, a µ opioid receptor antagonist, and the effect was confirmed for symptoms such as irritability (Non-patent Document 4). On the other hand, it has been reported by analysis of µ opioid receptor (MOR) knockout mice that ASD-like symptoms are induced by conversely a decrease in opioid signal (Non-patent Document 5). In addition, there is also a report that social behavior is increased by administering a MOR agonist in normal rats (Non-patent Document 6).

As described above, the possibility that the opioid signal is involved in ASD symptoms and social behavior has been suggested, but as discussed in Non-patent Document 5, which is a review article, the details thereof are unknown, and it is also unclear whether it is good to reduce or enhance the opioid signal in ASD treatment.

It is known that patients with human fragile X syndrome (FXS), which is caused by abnormalities of the X chromosome, present with social impairment symptoms, such as those often seen in ASD (Non-patent Documents 7 to 9).

Buprenorphine is a µ opioid receptor partial agonist and a κ opioid receptor antagonist. As a treatment agent for pain, dosage forms of a transdermal administration formulation, a transmucosal administration formulation (buccal, sublingual, suppository), and an injection (intravenous, intramuscular, subcutaneous) have been approved (Non-patent Documents 10 to 16. The contents of these documents are incorporated herein by reference).

Morphine is an opioid agonist, in particular a µ opioid receptor agonist. As a treatment agent for pain, dosage forms of an orally administered formulation, a transmucosally administered formulation (suppository), and an injection (intravenous, subcutaneous, etc.) have been approved (Non-patent Documents 17 to 22. The contents of these documents are incorporated herein by reference).

Patent Documents 1 and 2 and Non-patent Documents 23 to 25 do not describe or suggest the ASD treatment use of morphine or buprenorphine. Patent Document 3 describes combination data of oxytocin and naloxone (µ opioid receptor antagonist). Non-patent Document 26 describes pharmacokinetics when buprenorphine is sublingually administered. Non-patent Document 28 describes a result of evaluating the analgesic effect of buprenorphine using mice or the like.

### [PRIOR ART REFERENCES]

### [Patent Document]

[Patent Document 1] International Publication WO 2013/042054A1
[Patent Document 2] US 2019/0298703A1
[Patent Document 3] US 2019/0328830A1

### [Non-patent Document]

[Non-patent Document 1] JAMA Pediatr. 2014; 168 (8): 721-728.
[Non-patent Document 2] Pediatrics 2014; 133: e520-e529
[Non-patent Document 3] A neurochemical theory of autism. Trends Neurosci 2: 174-177.
[Non-patent Document 4] J Intellect Disabil Res. 2015 Apr; 59 (4): 293-306.
[Non-patent Document 5] Br J Pharmacol. 2018 Jul; 175 (14): 2750-2769.
[Non-patent Document 6] Vanderschuren LJ, Niesink RJ, Spruijt BM, Van Ree JM (1995b). Mu- and kappa-opioid receptor-mediated opioid effects on social play in juvenile rats. Eur J Pharmacol 276: 257-266.
[Non-patent Document 7] Howes et al., J Psychopharmacol (2018)
[Non-patent Document 8] RefBerry-Kravis et al., Nature Drug Dis (2018)
[Non-patent Document 9] Castagnola et al., Fron Synaptic Neurosci (2017)
[Non-patent Document 10] Butrans (registered trademark) US attachment (buprenorphine 5 mcg/h, 10 mcg/h, 20 mcg/h transdermal administration formulation)
[Non-patent Document 11] Norspan (registered trademark) tape 5 mg, 10 mg, 20 mg pharmaceutical product interview form (Japan) (buprenorphine 5 mg, 10 mg, 20 mg transdermal administration formulation)
[Non-patent Document 12] BELBUCA (registered trademark) US attachment (buprenorphine hydrochloride 75 mcg, 15 mcg, 300 mcg, 450 mcg, 600 mcg, 750 mcg, 900 mcg buccal film)
[Non-patent Document 13] SUBTEX (registered trademark) US attachment (buprenorphine 2 mg, 8 mg sublingual tablet)
[Non-patent Document 14] Temgesic 200 pg, 400 µg sublingual tablet attachment (buprenorphine hydrochloride)
[Non-patent Document 15] Lepetan (registered trademark) suppository 0.2 mg, 0.4 mg pharmaceutical product interview form (Japan) (buprenorphine hydrochloride)
[Non-patent Document 16] Lepetan (registered trademark) injection 0.2 mg, 0.4 mg pharmaceutical product interview form (Japan) (buprenorphine hydrochloride)
[Non-patent Document 17] INFUMORPH US attachment (morphine sulfate 200 mg/mL, 500 mg/mL injection)
[Non-patent Document 18] ARYMO (registered trademark) ER US attachment (morphine sulfate 15 mg, 30 mg, 60 mg sustained-release tablet)
[Non-patent Document 19] Morphine hydrochloride injectable 10 mg, 50 mg, 200 mg "TAKEDA" pharmaceutical product interview form (Japan)
[Non-patent Document 20] PACIF (registered trademark) capsule 30 mg, 60 mg, 120 mg Japanese attachment (morphine hydrochloride hydrate sustained-release capsule)
[Non-patent Document 21] Opso (registered trademark) oral liquid 5 mg, 10 mg Japanese attachment (liquid formulation for internal use of morphine hydrochloride)
[Non-patent Document 22] Anpec (registered trademark) suppository 10 mg, 20 mg, 30 mg Japanese attachment (morphine hydrochloride suppository)
[Non-patent Document 23] Social Cognitive and Affective Neuroscience, 2016, 1902-1909
[Non-patent Document 24] Psychoneuroendocrinology (2016) 63, 43-49
[Non-patent Document 25] Psychoneuroendocrinology (2015) 53, 10-15
[Non-patent Document 26] AmJ Psychiatry 2016; 173: 491-498; doi: 10.1176/appi.ajp.2015.15040535
[Non-patent Document 271 Br. J. clin. Pharmac. (1982), 13, 665-673
[Non-patent Document 28] Folia Pharmacologica Japonica (Folia Pharmcol. Japon.) 79, 147 to 162 (1982)

### [SUMMARY OF THE INVENTION]

### [PROBLEMS TO BE SOLVED BY THE INVENTION]

Provided are new pharmaceutical compositions and methods for the treatment and/or prevention of autism spectrum disorder, fragile X syndrome, autism spectrum disorder-like symptoms and/or disorders in social communication and/or social interaction, particularly preferably for the treatment of autism spectrum disorder.

### [MEANS FOR SOLVING THE PROBLEM]

The present inventors have extensively studied pharmaceutical compositions and methods for the treatment and/or prevention of autism spectrum disorder, fragile X syndrome, autism spectrum disorder-like symptoms and/or disorders in social communication and/or social interaction, particularly preferably for the treatment of autism spectrum disorder, and have found a suitable active ingredient. Furthermore, the present inventors have found a preferred administration method, dosage amount, and the like of the active ingredient.

The present invention relates to, for example, the following.

(1) A pharmaceutical composition for treatment and/or prevention of autism spectrum disorder, fragile X syndrome, and/or an autism spectrum disorder-like symptom, comprising buprenorphine or a pharmaceutically acceptable salt thereof and/or morphine or a pharmaceutically acceptable salt thereof.
(1A) A pharmaceutical composition for treating and/or preventing disorders in social communication and/or social interaction comprising buprenorphine or the pharmaceutically acceptable salt thereof and/or morphine or the pharmaceutically acceptable salt thereof.
(1B) A pharmaceutical composition for the treatment and/or prevention of autism spectrum disorder, fragile X syndrome, and/or the autism spectrum disorder-like symptom, comprising buprenorphine or a derivative thereof or the pharmaceutically acceptable salt thereof and/or morphine or a derivative thereof (Examples of derivatives of morphine include codeine) or the pharmaceutically acceptable salt thereof.
(1C) A pharmaceutical composition for the treatment and/or prevention of autism spectrum disorder, fragile X syndrome, and/or the autism spectrum disorder-like symptom, comprising buprenorphine, which is a µ opioid agonist, or the pharmaceutically acceptable salt thereof, and/or morphine or the pharmaceutically acceptable salt thereof.
(2) The pharmaceutical composition according to any of (1) and (1A) to (1C) above, for treatment and/or prevention of autism spectrum disorder.
(2A) The pharmaceutical composition according to any of (1) and (1A) to (1C) above, for treating and/or preventing a core symptom of autism spectrum disorder.
(3) The pharmaceutical composition according to any of (1), (2), (1A) to (1C), and (2A) above, for administration to a patient diagnosed with autism spectrum disorder.
(4) The pharmaceutical composition according to any of (1) to (3), (1A) to (1C), and (2A) above, for administering buprenorphine or the pharmaceutically acceptable salt thereof and/or morphine or the pharmaceutically acceptable salt thereof at a dose less than or equal to an effective amount for treating pain.
(5) The pharmaceutical composition according to any of (1) to (4), (1A) to (1C), and (2A) above, for administering buprenorphine or the pharmaceutically acceptable salt thereof and/or morphine or the pharmaceutically acceptable salt thereof in a dosage amount less than or equal to about 1/2 times of an effective amount for treating pain.
(5A) The pharmaceutical composition according to any of (1) to (5), (1A) to (1C), and (2A) above, for administering buprenorphine or the pharmaceutically acceptable salt thereof and/or morphine or the pharmaceutically acceptable salt thereof in a dosage amount less than or equal to about 1/10 times of an effective amount for treating pain.
(5B) The pharmaceutical composition according to any of (1) to (5), (1A) to (1C), (2A), and (5A) above, wherein the composition does not substantially exhibit an analgesic effect.
(5C) The pharmaceutical composition according to any of (1) to (5), (1A) to (1C), (2A), (5A), and (5B) above, wherein buprenorphine or the pharmaceutically acceptable salt thereof and/or morphine or the pharmaceutically acceptable salt thereof is administered in an effective amount for treating autism spectrum disorder.
(6) The pharmaceutical composition according to any of (1) to (5), (1A) to (1C), (2A), and (5A) to (5C) above, for administering buprenorphine or the pharmaceutically acceptable salt thereof and/or morphine or the pharmaceutically acceptable salt thereof in a dosage amount that is less than or equal to a minimum effective amount for treating pain.
(7) The pharmaceutical composition according to any of (1) to (6), (1A) to (1C), (2A), and (5A) to (5C) above, for administering buprenorphine or the pharmaceutically acceptable salt thereof and/or morphine or the pharmaceutically acceptable salt thereof in dosage amount of about 1/30 to about 3 times of the minimum effective amount for treating pain.
(7A) The pharmaceutical composition according to any of (1) to (7), (1A) to (1C), (2A), and (5A) to (5C) above, for administering buprenorphine or the pharmaceutically acceptable salt thereof and/or morphine or the pharmaceutically acceptable salt thereof in a dosage amount less than or equal to about 1/2 times of a minimum effective amount for treating pain.
(8) The pharmaceutical composition according to any of (1) to (7), (1A) to (1C), (2A), (5A) to (5C), and (7A) above, wherein buprenorphine or the pharmaceutically acceptable salt thereof and/or morphine or the pharmaceutically acceptable salt thereof is buprenorphine or the pharmaceutically acceptable salt thereof.
(9) The pharmaceutical composition according to any of (1) to (8), (1A) to (1C), (2A), (5A) to (5C), and (7A) above, wherein the pharmaceutical composition provides a maximum plasma concentration of buprenorphine or the pharmaceutically acceptable salt thereof of about 1 ng/mL or less.
(10) The pharmaceutical composition according to any of (1) to (9), (1A) to (1C), (2A), (5A) to (5C), and (7A) above, wherein the pharmaceutical composition provides a maximum plasma concentration of buprenorphine or the pharmaceutically acceptable salt thereof of about 4 pg/mL to about 0.6 ng/mL.
(10A) The pharmaceutical composition according to any of (1) to (10), (1A) to (1C), (2A), (5A) to (5C), and (7A) above, wherein the pharmaceutical composition provides a maximum plasma concentration of buprenorphine or the pharmaceutically acceptable salt thereof of more than 3.3 pg/mL and less than or equal to about 0.6 ng/mL.
(10B) The pharmaceutical composition according to any of (1) to (10), (1A) to (1C), (2A), (5A) to (5C), (7A), and (10A) above, wherein the pharmaceutical composition provides a maximum plasma concentration of buprenorphine or the pharmaceutically acceptable salt thereof of about 0.6 ng/mL or less.
(10C) The pharmaceutical composition according to any of (1) to (10), (1A) to (1C), (2A), (5A) to (5C), (7A), (10A), and (10B) above, wherein the pharmaceutical composition provides a maximum plasma concentration of buprenorphine or the pharmaceutically acceptable salt thereof of about 1 pg/mL to about 1 ng/mL.
(10D) The pharmaceutical composition according to any of (1) to (10), (1A) to (1C), (2A), (5A) to (5C), (7A), and (10A) to (10C) above, wherein the pharmaceutical composition provides a plasma concentration of buprenorphine in the range of about 4 pg/mL to about 1 ng/mL (e.g., about 4 pg/mL to about 0.6 ng/mL) (e.g., continuously for about 30 minutes or more).
(10E) The pharmaceutical composition according to any of (1) to (10), (1A) to (1C), (2A), (5A) to (5C), (7A), and (10A) to (10D) above, wherein the pharmaceutical composition provides a plasma concentration of buprenorphine in the range of about 4 pg/mL to about 1 ng/mL (e.g., about 3 pg/mL to about 0.6 ng/mL).
(11) The pharmaceutical composition according to any of (1) to (10), (1A) to (1C), (2A), (5A) to (5C), (7A), and (10A) to (10E) above, wherein the pharmaceutical composition provides a maximum plasma concentration of buprenorphine or the pharmaceutically acceptable salt thereof of about 0.2 ng/mL or less.
(12) The pharmaceutical composition according to any of (1) to (11), (1A) to (1C), (2A), (5A) to (5C), (7A), and (10A) to (10E) above, wherein the pharmaceutical composition provides a maximum plasma concentration of buprenorphine or the pharmaceutically acceptable salt thereof of about 8 pg/mL to about 0.2 ng/mL.
(12A) The pharmaceutical composition according to any of (1) to (12), (1A) to (1C), (2A), (5A) to (5C), (7A), and (10A) to (10E) above, wherein the maximum plasma concentration or plasma concentration is the maximum plasma concentration or plasma concentration at a steady state.
(13) The pharmaceutical composition according to any of (1) to (12), (1A) to (1C), (2A), (5A) to (5C), (7A), (10A) to (10E), and (12A) above,
   wherein the pharmaceutical composition is any of the following a) to c):
   a) a pharmaceutical composition for transdermal administration comprising about 0.01 mg to about 10 mg of buprenorphine or the pharmaceutically acceptable salt thereof;
   b) a pharmaceutical composition for transdermal administration that releases buprenorphine or the pharmaceutically acceptable salt thereof at about 0.01 µg/h to about 10 pg/h; and
   c) a pharmaceutical composition for transmucosal administration comprising about 0.0001 mg to 0.1 mg of buprenorphine or the pharmaceutically acceptable salt thereof.
(13A) The pharmaceutical composition according to any of (1) to (13), (1A) to (1C), (2A), (5A) to (5C), (7A), (10A) to (10E), and (12A) above,
   wherein the pharmaceutical composition is any of the following a) to c):
   a) a pharmaceutical composition for transdermal administration comprising about 0.01 mg to about 5 mg of buprenorphine or the pharmaceutically acceptable salt thereof;
   b) a pharmaceutical composition for transdermal administration that releases buprenorphine or the pharmaceutically acceptable salt thereof at about 0.1 µg/h to about 5 pg/h; and
   c) a pharmaceutical composition for transmucosal administration comprising about 0.001 mg to 0.075 mg of buprenorphine or the pharmaceutically acceptable salt thereof.
(13B) The pharmaceutical composition according to any of (1) to (13), (1A) to (1C), (2A), (5A) to (5C), (7A), (10A) to (10E), (12A), and (13A) above, wherein the pharmaceutical composition is either
   a) a pharmaceutical composition for transdermal administration comprising about 0.56 mg to about 1.7 mg of buprenorphine or the pharmaceutically acceptable salt thereof, or
   b) a pharmaceutical composition for transdermal administration that releases buprenorphine or the pharmaceutically acceptable salt thereof at about 0.56 µg/h to about 1.7 µg/h.
(13C) The pharmaceutical composition according to any of (1) to (13), (1A) to (1C), (2A), (5A) to (5C), (7A), (10A) to (10E), (12A), (13A), and (13B) above, wherein the pharmaceutical composition is either
   a) a pharmaceutical composition for transdermal administration comprising about 0.56 mg or about 1.7 mg of buprenorphine or the pharmaceutically acceptable salt thereof, or
   b) a pharmaceutical composition for transdermal administration that releases buprenorphine or the pharmaceutically acceptable salt thereof at about 0.56 µg/h or about 1.7 µg/h.
(14) The pharmaceutical composition according to any of (1) to (13), (1A) to (1C), (2A), (5A) to (5C), (7A), (10A) to (10E), (12A), and (13A) to (13C) above, for administration at an administration interval of 12 hours or more.
(14A) The pharmaceutical composition according to any of (1) to (14), (1A) to (1C), (2A), (5A) to (5C), (7A), (10A) to (10E), (12A), and (13A) to (13C) above, wherein the number of doses is twice or less per day.
(15) The pharmaceutical composition according to any of (1) to (14), (1A) to (1C), (2A), (5A) to (5C), (7A), (10A) to (10E), (12A), (13A) to (13C), and (14A) above, wherein the drug efficacy lasts for 12 hours or more after administration.
(16) The pharmaceutical composition according to any of (1) to (15), (1A) to (1C), (2A), (5A) to (5C), (7A), (10A) to (10E), (12A), (13A) to (13C), and (14A) above, for administering buprenorphine or the pharmaceutically acceptable salt thereof without combination use with other drugs.
(16A) The pharmaceutical composition according to any of (1) to (16), (1A) to (1C), (2A), (5A) to (5C), (7A), (10A) to (10E), (12A), (13A) to (13C), and (14A) above, which is a pharmaceutical composition for transdermal administration or a pharmaceutical composition for transmucosal administration.
(16B) The pharmaceutical composition according to any of (1) to (16), (1A) to (1C), (2A), (5A) to (5C), (10A) to (10E), (12A), (13A) to (13C), and (14A) above, which is a pharmaceutical composition for transdermal administration.
(16C) The pharmaceutical composition according to any of (1) to (16), (1A) to (1C), (2A), (5A) to (5C), (7A), (10A) to (10E), (12A), (13A) to (13C), and (14A) above, which is a pharmaceutical composition for transmucosal administration.
(17) The pharmaceutical composition according to any of (1) to (7), (1A) to (1C), (2A), (5A) to (5C), and (7A) above, wherein buprenorphine or the pharmaceutically acceptable salt thereof and/or morphine or the pharmaceutically acceptable salt thereof is morphine or the pharmaceutically acceptable salt thereof.
(18) The pharmaceutical composition according to (17) above, wherein the pharmaceutical composition provides a maximum plasma concentration of morphine or pharmaceutically acceptable salt thereof of about 200 ng/mL or less.
(19) The pharmaceutical composition according to (17) or (18) above, wherein the pharmaceutical composition provides a maximum plasma concentration of morphine or the pharmaceutically acceptable salt thereof of about 1 ng/mL to about 200 ng/mL.
(19A) The pharmaceutical composition according to any of (1) to (19), (1A) to (1C), (2A), (5A) to (5C), (7A), (10A) to (10E), (12A), (13A) to (13C), (14A), and (16A) to (16C) above, wherein the pharmaceutical composition activates a nucleus accumbens and medial prefrontal cortex, and does not activate dorsomedial periaqueductal gray.
(101) A method for the treatment and/or prevention of autism spectrum disorder, fragile X syndrome, and/or the autism spectrum disorder-like symptom, comprising administering to a patient in need thereof buprenorphine or the pharmaceutically acceptable salt thereof and/or morphine or the pharmaceutically acceptable salt thereof.
(101A) A method of treating and/or preventing disorders in social communication and/or social interaction comprising administering buprenorphine or the pharmaceutically acceptable salt thereof and/or morphine or the pharmaceutically acceptable salt thereof.
(101B) A method of treating and/or preventing autism spectrum disorder, fragile X syndrome, and/or the autism spectrum disorder-like symptom, comprising administering buprenorphine or the derivative thereof or the pharmaceutically acceptable salt thereof and/or morphine or the derivative thereof (Examples of derivatives of morphine include codeine) or the pharmaceutically acceptable salt thereof.
(101C) A method of treating and/or preventing autism spectrum disorder, fragile X syndrome, and/or autism spectrum disorder-like symptom comprising administering buprenorphine, which is a µ opioid agonist, or the pharmaceutically acceptable salt thereof, and/or morphine or the pharmaceutically acceptable salt thereof.
(102) The method according to any of (101) and (101A) to (101C) above, for treatment and/or prevention of autism spectrum disorder.
(102A) The method according to any of (101) and (101A) to (101C) above, for treating and/or preventing a core symptom of autism spectrum disorder.
(103) The method according to any of (101), (102), (101A) to (101C), and (102A) above, for administration to a patient diagnosed with autism spectrum disorder.
(104) The method according to any of (101) to (103), (101A) to (101C), and (102A) above, comprising administering buprenorphine or the pharmaceutically acceptable salt thereof and/or morphine or the pharmaceutically acceptable salt thereof at a dose less than or equal to an effective amount for treating pain.
(105) The method according to any of (101) to (104), (101A) to (101C), and (102A) above, comprising administering buprenorphine or the pharmaceutically acceptable salt thereof and/or morphine or the pharmaceutically acceptable salt thereof in a dosage amount less than or equal to about 1/2 times of an effective amount for treating pain.
(105A) The method according to any of (101) to (105), (101A) to (101C), and (102A) above, comprising administering buprenorphine or the pharmaceutically acceptable salt thereof and/or morphine or the pharmaceutically acceptable salt thereof in a dosage amount less than or equal to about 1/10 times of an effective amount for treating pain.
(105B) The method according to any of (101) to (105), (101A) to (101C), (102A), and (105A) above, wherein the method does not substantially exhibit an analgesic effect.
(105C) The method according to any of (101) to (105), (101A) to (101C), (102A), (105A), and (105B) above, wherein buprenorphine or the pharmaceutically acceptable salt thereof and/or morphine or the pharmaceutically acceptable salt thereof is administered in an effective amount for treating autism spectrum disorder.
(106) The method according to any of (101) to (105), (101A) to (101C), (102A), and (105A) to (105C) above, wherein buprenorphine or the pharmaceutically acceptable salt thereof and/or morphine or the pharmaceutically acceptable salt thereof is administered in a dosage amount that is less than or equal to a minimum effective amount for treating pain.
(107) The method according to any of (101) to (106), (101A) to (101C), (102A), and (105A) to (105C) above, wherein buprenorphine or the pharmaceutically acceptable salt thereof and/or morphine or the pharmaceutically acceptable salt thereof is administered in dosage amount of about 1/30 to about 3 times of the minimum effective amount for treating pain.
(107A) The method according to any of (101) to (107), (101A) to (101C), (102A), and (105A) to (105C) above, for administering buprenorphine or the pharmaceutically acceptable salt thereof and/or morphine or the pharmaceutically acceptable salt thereof in a dosage amount less than or equal to about 1/2 times of a minimum effective amount for treating pain.
(108) The method according to any of (101) to (107), (101A) to (101C), (102A), (105A) to (105C), and (107A) above, wherein buprenorphine or the pharmaceutically acceptable salt thereof and/or morphine or the pharmaceutically acceptable salt thereof is buprenorphine or the pharmaceutically acceptable salt thereof.
(109) The method according to any of (101) to (108), (101A) to (101C), (102A), (105A) to (105C), and (107A) above, wherein the maximum plasma concentration of buprenorphine or the pharmaceutically acceptable salt thereof in the administered patient is less than or equal to about 1 ng/mL.
(110) The method according to any of (101) to (109), (101A) to (101C), (102A), (105A) to (105C), and (107A) above, wherein the maximum plasma concentration of buprenorphine or the pharmaceutically acceptable salt thereof in the administered patient is about 4 pg/mL to about 0.6 ng/mL.
(110A) The method according to any of (101) to (110), (101A) to (101C), (102A), (105A) to (105C), and (107A) above, wherein the maximum plasma concentration of buprenorphine or the pharmaceutically acceptable salt thereof in the patient administered is more than 3.3 pg/mL and less than or equal to about 0.6 ng/mL.
(110B) The method according to any of (101) to (110), (101A) to (101C), (102A), (105A) to (105C), (107A), and (110A) above, wherein the maximum plasma concentration of buprenorphine or the pharmaceutically acceptable salt thereof in the administered patient is less than or equal to about 0.6 ng/mL.
(110C) The method according to any of (101) to (110), (101A) to (101C), (102A), (105A) to (105C), (107A) (110A), and (110B) above, wherein the maximum plasma concentration of buprenorphine or the pharmaceutically acceptable salt thereof in the administered patient is about 1 pg/mL to about 1 ng/mL.
(110D) The method according to any of (101) to (110), (101A) to (101C), (102A), (105A) to (105C), (107A), and (110A) to (110C) above, wherein the maximum plasma concentration of buprenorphine or the pharmaceutically acceptable salt thereof in the administered patient is in the range of about 4 pg/mL to about 1 ng/mL (e.g., about 4 pg/mL to about 0.6 ng/mL) (e.g., for about 30 minutes or more in a row).
(110E) The method according to any of (101) to (110), (101A) to (101C), (102A), (105A) to (105C), (107A) and (110A) to (110 D) above, wherein the maximum plasma concentration of buprenorphine or the pharmaceutically acceptable salt thereof in the administered patient is in the range of about 4 pg/mL to about 1 ng/mL (e.g., about 3 pg/mL to about 0.6 ng/mL).
(111) The method according to any of (101) to (110), (101A) to (101C), (102A), (105A) to (105C), (107A), and (110A) to (110E) above, wherein the maximum plasma concentration of buprenorphine or the pharmaceutically acceptable salt thereof in the administered patient is about 0.2 ng/mL or less.
(112) The method according to any of (101) to (111), (101A) to (101C), (102A), (105A) to (105C), (107A), and (110A) to (110E) above, wherein the maximum plasma concentration of buprenorphine or the pharmaceutically acceptable salt thereof in the administered patient is about 8 pg/mL to about 0.2 ng/mL.
(112A) The method according to any of (101) to (112), (101A) to (101C), (102A), (105A) to (105C), (107A), and (110A) to (110E) above, wherein the maximum plasma concentration or plasma concentration is the maximum plasma concentration or plasma concentration at a steady state.
(113) The method according to any of (101) to (112), (101A) to (101C), (102A), (105A) to (105C), (107A), (110A) to (110E), and (112A) above,
   wherein the method includes any of the following a) to c):
   a) administering transdermally about 0.01 mg to about 10 mg of buprenorphine or the pharmaceutically acceptable salt thereof;
   b) transdermally administering buprenorphine or the pharmaceutically acceptable salt thereof at about 0.01 µg/h to about 10 pg/h; and
   c) administering about 0.0001 mg to 0.1 mg transmucosally of buprenorphine or the pharmaceutically acceptable salt thereof.
(113A) The method according to any of (101) to (113), (101A) to (101C), (102A), (105A) to (105C), (107A), (110A) to (110E), and (112A) above,
   wherein the method includes any of the following a) to c):
   a) administering transdermally about 0.01 mg to about 5 mg of buprenorphine or the pharmaceutically acceptable salt thereof;
   b) transdermally administering buprenorphine or the pharmaceutically acceptable salt thereof at about 0.1 µg/h to about 5 µg/h; and
   c) administering 0.001 mg to 0.075 mg of buprenorphine or the pharmaceutically acceptable salt thereof transmucosally.
(113B) The method according to any of (101) to (113), (101A) to (101C), (102A), (105A) to (105C), (107A), (110A) to (110E), (112A), and (113A) above, wherein the method is either
   a) transdermally administering about 0.56 mg to about 1.7 mg of buprenorphine or the pharmaceutically acceptable salt thereof, or
   b) transdermally administering at about 0.56 µg/h to about 1.7 µg/h of buprenorphine or the pharmaceutically acceptable salt thereof.
(113C) The method according to any of (101) to (113), (101A) to (101C), (102A), (105A) to (105C), (107A), (110A) to (110E), (112A), (113A), and (113B) above, wherein the method is either
   a) administering transdermally about 0.56 mg or about 1.7 mg of buprenorphine or the pharmaceutically acceptable salt thereof, or
   b) administering transdermally at about 0.56 µg/h or about 1.7 µg/h of buprenorphine or the pharmaceutically acceptable salt thereof.
(114) The method according to any of (101) to (113), (101A) to (101C), (102A), (105A) to (105C), (107A), (110A) to (110E), (112A) above, and (113A) to (113C) above, wherein buprenorphine or the pharmaceutically acceptable salt thereof is administered at an administration interval of 12 hours or more.
(114A) The method according to any of (101) to (114), (101A) to (101C), (102A), (105A) to (105C), (107A), (110A) to (110E), (112A) above, and (113A) to (113C) above, wherein the number of doses is twice or less per day.
(115) The method according to any of (101) to (114), (101A) to (101C), (102A), (105A) to (105C), (107A), (110A) to (110E), (112A), (113A) to (113C), and (114A) above, wherein the drug efficacy lasts for 12 hours or more after administration.
(116) The method according to any of (101) to (115), (101A) to (101C), (102A), (105A) to (105C), (107A), (110A) to (110E), (112A), (113A) to (113C), and (114A) above, for administering buprenorphine or the pharmaceutically acceptable salt thereof without combination use with other drugs.
(116A) The method according to any of (101) to (116), (101A) to (101C), (102A), (105A) to (105C), (107A), (110A) to (110E), (112A), (113A) to (113C), and (114A) above, wherein buprenorphine or the pharmaceutically acceptable salt thereof is transdermally or transmucosally administered.
(116B) The method according to any of (101) to (116), (101A) to (101C), (102A), (105A) to (105C), (107A), (110A) to (110E), (112A), (113A) to (113C), and (114A) above, wherein buprenorphine or the pharmaceutically acceptable salt thereof is transdermally administered.
(116C) The method according to any of (101) to (116), (101A) to (101C), (102A), (105A) to (105C), (107A), (110A) to (110E), (112A), (113A) to (113C), and (114A) above, wherein buprenorphine or the pharmaceutically acceptable salt thereof is administered transmucosally.
(117) The method according to any of (101) to (107), (101A) to (101C), (102A), (105A) to (105C), and (107A) above, wherein buprenorphine or the pharmaceutically acceptable salt thereof and/or morphine or the pharmaceutically acceptable salt thereof is morphine or the pharmaceutically acceptable salt thereof.
(118) The method according to (117) above, wherein the maximum plasma concentration of morphine or the pharmaceutically acceptable salt thereof is about 200 ng/mL or less.
(119) The method according to (117) or (118) above, wherein the maximum plasma concentration of morphine or the pharmaceutically acceptable salt thereof is about 1 ng/mL to about 200 ng/mL.
(119A) The method according to any of (101) to (119), (101A) to (101C), (102A), (105A) to (105C), (107A), (110A) to (110E), (112A), (113A) to (113C), (114A), and (116A) to (116C) above, wherein the pharmaceutical composition activates a nucleus accumbens and medial prefrontal cortex, and does not activate dorsomedial periaqueductal gray.
(201) Buprenorphine or the pharmaceutically acceptable salt thereof and/or morphine or the pharmaceutically acceptable salt thereof for use in the treatment and/or prevention of autism spectrum disorder, fragile X syndrome, and/or the autism spectrum disorder-like symptom.
(201A) Buprenorphine or the pharmaceutically acceptable salt thereof and/or morphine or the pharmaceutically acceptable salt thereof for use in the treatment and/or prevention of disorders in social communication and/or social interaction.
(201B) Buprenorphine or the derivative thereof or the pharmaceutically acceptable salt thereof and/or morphine or the derivative thereof (Examples of derivatives of morphine include codeine) or the pharmaceutically acceptable salt thereof, for the treatment and/or prevention of autism spectrum disorder, fragile X syndrome, and/or the autism spectrum disorder-like symptom.
(201C) The µ opioid agonist buprenorphine or the pharmaceutically acceptable salt thereof and/or morphine or the pharmaceutically acceptable salt thereof for the treatment and/or prevention of autism spectrum disorder, fragile X syndrome, and/or the autism spectrum disorder-like symptom.
(202) The buprenorphine or the pharmaceutically acceptable salt thereof and/or morphine or the pharmaceutically acceptable salt thereof according to any of (201) and (201A) to (201C) above for treatment and/or prevention of autism spectrum disorder.
(202A) The buprenorphine or the pharmaceutically acceptable salt thereof and/or morphine or the pharmaceutically acceptable salt thereof according to any of (201) and (201A) to (201C) above for treatment and/or prevention of a core symptom of autism spectrum disorder.
(203) The buprenorphine or the pharmaceutically acceptable salt thereof and/or morphine or the pharmaceutically acceptable salt thereof according to any of (201), (202), (201A) to (201C), and (202A) above, for administration to a patient diagnosed with autism spectrum disorder.
   The buprenorphine or the pharmaceutically acceptable salt thereof and/or morphine or the pharmaceutically acceptable salt thereof according to any of (201) to (203), (201A) to (201C), and (202A) above, comprising one or more features according to any one or more of (1) to (19), (1A) to (1C), (2A), (5A) to (5C), (7A), (10A) to (10E), (12A), (13A) to (13C), (14A), (16A) to (16C), (101) to (119), (101A) to (101C), (102A), (105A) to (105C), (107A), (110A) to (110E), (112A), (113A) to (113C), (114A), and (116A) to (116C) above.
(301) Use of buprenorphine or the pharmaceutically acceptable salt thereof and/or morphine or the pharmaceutically acceptable salt thereof, for the production of a pharmaceutical composition for the treatment and/or prevention of autism spectrum disorder, fragile X syndrome, and/or the autism spectrum disorder-like symptom.
(301A) Use of buprenorphine or the pharmaceutically acceptable salt thereof and/or morphine or the pharmaceutically acceptable salt thereof, for the production of a pharmaceutical composition for the treatment and/or prevention of disorders in social communication and/or social interaction.
(301B) Use of buprenorphine or the derivative thereof or the pharmaceutically acceptable salt thereof and/or morphine or the derivative thereof (Examples of derivatives of morphine include codeine) or the pharmaceutically acceptable salt thereof, for the production of a pharmaceutical composition for the treatment and/or prevention of autism spectrum disorder, fragile X syndrome, and/or the autism spectrum disorder-like symptom.
(301C) Use of buprenorphine, which is a µ opioid agonist, or the pharmaceutically acceptable salt thereof, and/or morphine or the pharmaceutically acceptable salt thereof, for the production of a pharmaceutical composition for the treatment and/or prevention of autism spectrum disorder, fragile X syndrome, and/or the autism spectrum disorder-like symptom.
(302) The use according to any of (301) and (301A) to (301C) above, for producing a pharmaceutical composition for treatment and/or prevention of autism spectrum disorder.
(302A) The use according to any of (301) and (301A) to (301C) above for producing a pharmaceutical composition for treatment and/or prevention of a core symptom of autism spectrum disorder.
(303) The use according to any of (301), (302), (301A) to (301C), and (302A) above for producing a pharmaceutical composition for administration to a patient diagnosed with autism spectrum disorder.
(304) The use according to any of (301) to (303), (301A) to (301C), and (302A) above, for producing a pharmaceutical composition wherein a content of buprenorphine or the pharmaceutically acceptable salt thereof and/or morphine or the pharmaceutically acceptable salt thereof is an effective amount for treating pain or less.
(305) The use according to any of (301) to (304), (301A) to (301C), and (302A) above, for producing a pharmaceutical composition wherein a content of buprenorphine or the pharmaceutically acceptable salt thereof and/or morphine or the pharmaceutically acceptable salt thereof is less than or equal to about 1/2 times of an effective amount for treating pain.
(305A) The use according to any of (301) to (305), (301A) to (301C), and (302A) above, for producing a pharmaceutical composition wherein a content of buprenorphine or the pharmaceutically acceptable salt thereof and/or morphine or the pharmaceutically acceptable salt thereof is less than or equal to about 1/10 times of an effective amount for treating pain.
(305B) The use according to any of (301) to (305), (301A) to (301C), (302A), and (305A) above, wherein the pharmaceutical composition does not substantially exhibit an analgesic effect.
(305C) The use according to any of (301) to (305), (301A) to (301C), (302A), (305A), and (305B) above, for producing a pharmaceutical composition wherein a content of buprenorphine or the pharmaceutically acceptable salt thereof and/or morphine or the pharmaceutically acceptable salt thereof is an effective amount for treating autism spectrum disorder.
(306) The use according to any of (301) to (305), (301A) to (301C), (302A), and (305A) to (305C) above, for producing a pharmaceutical composition wherein a content of buprenorphine or the pharmaceutically acceptable salt thereof and/or morphine or the pharmaceutically acceptable salt thereof is less than or equal to a minimum effective amount for treating pain.
(307) The use according to any of (301) to (306), (301A) to (301C), (302A), and (305A) to (305C) above, for the production of a pharmaceutical composition wherein the content of buprenorphine or the pharmaceutically acceptable salt thereof and/or morphine or the pharmaceutically acceptable salt thereof is about 1/30 to about 3 times of the minimum effective amount for treating pain.
(307A) The use according to any of (301) to (307), (301A) to (301C), (302A), and (305A) to (305C) above, for producing a pharmaceutical composition wherein a content of buprenorphine or the pharmaceutically acceptable salt thereof and/or morphine or the pharmaceutically acceptable salt thereof is less than or equal to about 1/2 times of the minimum effective amount for treating pain.
(308) The use according to any of (301) to (307), (301A) to (301C), (302A), (305A) to (305C), and (307A) above, wherein buprenorphine or the pharmaceutically acceptable salt thereof and/or morphine or the pharmaceutically acceptable salt thereof is buprenorphine or the pharmaceutically acceptable salt thereof.
(309) The use according to any of (301) to (308), (301A) to (301C), (302A), (305A) to (305C), and (307A) above, wherein the pharmaceutical composition is a pharmaceutical composition provides a maximum plasma concentration of buprenorphine or the pharmaceutically acceptable salt thereof of about 1 ng/mL or less.
(310) The use according to any of (301) to (309), (301A) to (301C), (302A), (305A) to (305C), and (307A) above, wherein the pharmaceutical composition provides a maximum plasma concentration of buprenorphine or the pharmaceutically acceptable salt thereof of about 4 pg/mL to about 0.6 ng/mL.
(310A) The use according to any of (301) to (310), (301A) to (301C), (302A), (305A) to (305C), and (307A) above, wherein the pharmaceutical composition is a pharmaceutical composition provides a maximum plasma concentration of buprenorphine or the pharmaceutically acceptable salt thereof of more than 3.3 pg/mL and less than or equal to about 0.6 ng/mL.
(310B) The use according to any of (301) to (310), (301A) to (301C), (302A), (305A) to (305C), (307A), and (310A) above, wherein the pharmaceutical composition is a pharmaceutical composition that provides a maximum plasma concentration of buprenorphine or the pharmaceutically acceptable salt thereof of about 0.6 ng/mL or less.
(310C) The use according to any of (301) to (310), (301A) to (301C), (302A), (305A) to (305C), (307A), (310A), and (310B) above, wherein the pharmaceutical composition is a pharmaceutical composition that provides a maximum plasma concentration of buprenorphine or the pharmaceutically acceptable salt thereof of about 1 pg/mL to about 1 ng/mL.
(310D) The use according to any of (301) to (310), (301A) to (301C), (302A), (305A) to (305C), and (310A) to (310C) above, wherein the pharmaceutical composition is a pharmaceutical composition provides a plasma concentration of buprenorphine in the range of about 4 pg/mL to about 1 ng/mL (e.g., about 4 pg/mL to about 0.6 ng/mL) (e.g., continuously for about 30 minutes or more).
(310E) The use according to any of (301) to (310), (301A) to (301C), (302A), (305A) to (305C), (307A), and (310A) to (310D) above, wherein the pharmaceutical composition is a pharmaceutical composition provides a plasma concentration of buprenorphine in the range of about 4 pg/mL to about 1 ng/mL (e.g., about 3 pg/mL to about 0.6 ng/mL).
(311) The use according to any of (301) to (310), (301A) to (301C), (302A), (305A) to (305C), (307A), and (310A) to (310E) above, wherein the pharmaceutical composition is a pharmaceutical composition that provides a maximum plasma concentration of buprenorphine or the pharmaceutically acceptable salt thereof of about 0.2 ng/mL or less.
(312) The use according to any of (301) to (311), (301A) to (301C), (302A), (305A) to (305C), (307A), and (310A) to (310E) above, wherein the pharmaceutical composition is a pharmaceutical composition that provides a maximum plasma concentration of buprenorphine or the pharmaceutically acceptable salt thereof of about 8 pg/mL to about 0.2 ng/mL.
   (312A) The use according to any of (301) to (312), (301A) to (301C), (302A), (305A) to (305C), (307A), and (310A) to (310E) above, wherein the maximum plasma concentration or plasma concentration is the maximum plasma concentration or plasma concentration at a steady state.
(313) The use according to any of (301) to (312), (301A) to (301C), (302A), (305A) to (305C), (307A), (310A) to (310E), and (312A) above,
   wherein the pharmaceutical composition is any of the following a) to c):
   a) a pharmaceutical composition for transdermal administration comprising about 0.01 mg to about 10 mg of buprenorphine or the pharmaceutically acceptable salt thereof;
   b) a pharmaceutical composition for transdermal administration that releases buprenorphine or the pharmaceutically acceptable salt thereof at about 0.01 µg/h to about 10 pg/h; and
   c) a pharmaceutical composition for transmucosal administration comprising about 0.0001 mg to 0.1 mg of buprenorphine or the pharmaceutically acceptable salt thereof.
(313A) The use according to any of (301) to (313), (301A) to (301C), (302A), (305A) to (305C), (307A), (310A) to (310E), and (312A) above,
   wherein the pharmaceutical composition is any of the following a) to c):
   a) a pharmaceutical composition for transdermal administration comprising about 0.01 mg to about 5 mg of buprenorphine or the pharmaceutically acceptable salt thereof;
   b) a pharmaceutical composition for transdermal administration that releases buprenorphine or the pharmaceutically acceptable salt thereof at about 0.1 µg/h to about 5 µg/h; and
   c) a pharmaceutical composition for transmucosal administration comprising about 0.001 mg to 0.075 mg of buprenorphine or the pharmaceutically acceptable salt thereof.
(313B) The use according to any of (301) to (313), (301A) to (301C), (302A), (305A) to (305C), (307A), (310A) to (310E), (312A), and (313A) above,
   wherein the pharmaceutical composition is either
   a) a pharmaceutical composition for transdermal administration comprising about 0.56 mg to about 1.7 mg of buprenorphine or the pharmaceutically acceptable salt thereof, or
   b) a pharmaceutical composition for transdermal administration that releases buprenorphine or the pharmaceutically acceptable salt thereof at about 0.56 µg/h to about 1.7 µg/h.
(313C) The use according to any of (301) to (313), (301A) to (301C), (302A), (305A) to (305C), (307A), (310A) to (310E), (312A), (313A), and (313B) above,
   wherein the pharmaceutical composition is either
   a) a pharmaceutical composition for transdermal administration comprising about 0.56 mg or about 1.7 mg of buprenorphine or the pharmaceutically acceptable salt thereof, or
   b) a pharmaceutical composition for transdermal administration that releases buprenorphine or the pharmaceutically acceptable salt thereof at about 0.56 µg/h or about 1.7 µg/h.
(314) The use according to any of (301) to (313), (301A) to (301C), (302A), (305A) to (305C), (307A), (310A) to (310E), (312A), and (313A) to (313C) above, wherein the pharmaceutical composition is a pharmaceutical composition for administration at an administration interval of 12 hours or more.
(314A) The use according to any of (301) to (314), (301A) to (301C), (302A), (305A) to (305C), (307A), (310A) to (310E), (312A), and (313A) to (313C) above, wherein the pharmaceutical composition is a pharmaceutical composition for administration twice or less per day.
(315) The pharmaceutical composition according to any of (301) to (314), (301A) to (301C), (302A), (305A) to (305C), (307A), (310A) to (310E), (312A), (313A) to (313C), and (314A) above, wherein the pharmaceutical composition is a pharmaceutical composition whose drug efficacy lasts for 12 hours or more after administration.
(316) The use according to any of (301) to (315), (301A) to (301C), (302A), (305A) to (305C), (307A), (310A) to (310E), (312A), (313A) to (313C), and (314A) above, wherein the pharmaceutical composition is a pharmaceutical composition comprising only buprenorphine or the pharmaceutically acceptable salt thereof as an active ingredient.
(316A) The use according to any of (301) to (316), (301A) to (301C), (302A), (305A) to (305C), (307A), (310A) to (310E), (312A), (313A) to (313C), and (314A) above, wherein the pharmaceutical composition is a pharmaceutical composition for transdermal administration or a pharmaceutical composition for transmucosal administration.
(316B) The use according to any of (301) to (316), (301A) to (301C), (302A), (305A) to (305C), (307A), (310A) to (310E), (312A), (313A) to (313C), and (314A) above, wherein the pharmaceutical composition is a pharmaceutical composition for transdermal administration.
(316C) The use according to any of (301) to (316), (301A) to (301C), (302A), (305A) to (305C), (307A), (310A) to (310E), (312A), (313A) to (313C), and (314A) above, wherein the pharmaceutical composition is a pharmaceutical composition for transmucosal administration.
(317) The use according to any of (301) to (307), (301A) to (301C), (302A), (305A) to (305C), and (307A) above, wherein buprenorphine or the pharmaceutically acceptable salt thereof and/or morphine or the pharmaceutically acceptable salt thereof is morphine or the pharmaceutically acceptable salt thereof.
(318) The use according to (317) above, wherein the pharmaceutical composition is a pharmaceutical composition providing a maximum plasma concentration of morphine or pharmaceutically acceptable salt thereof of about 200 ng/mL or less.
(319) The use according to (317) or (318) above, wherein the pharmaceutical composition is a pharmaceutical composition providing a maximum plasma concentration of morphine or the pharmaceutically acceptable salt thereof of about 1 ng/mL to about 200 ng/mL.
(319A) The use according to any of (301) to (319), (301A) to (301C), (302A), (305A) to (305C), (307A), (310A) to (310E), (312A), (313A) to (313C), (314A), and (316A) to (316C) above, wherein the pharmaceutical composition is a pharmaceutical composition for activating a nucleus accumbens and medial prefrontal cortex and not activating dorsomedial periaqueductal gray.

Use of buprenorphine or the pharmaceutically acceptable salt thereof and/or morphine or the pharmaceutically acceptable salt thereof, comprising one or more of the features according to any one or more of the above groups of items.

### [EFFECT OF THE INVENTION]

The pharmaceutical compositions and methods of the present invention are useful for the treatment and/or prevention of various psychiatric diseases, in particular autism spectrum disorder, fragile X syndrome, the autism spectrum disorder-like symptom, and/or disorders in social communication and/or social interaction.

### [BRIEF DESCRIPTION OF THE DRAWINGS]

Fig. 1 shows the results of evaluating the improvement effect on sociality of morphine using valproic acid model mice which are ASD model mice.
Fig. 2 shows the results of evaluating the improvement effect on sociality of buprenorphine using valproic acid model mice which are ASD model mice.
Fig. 3 shows the results of evaluating the improvement effect on sociality of tramadol using valproic acid model mice which are ASD model mice.
Fig. 4 shows the relationship between the plasma concentration of an unchanged form of morphine, improvement in social impairment, and analgesic drug efficacy.
Fig. 5 shows the relationship between the plasma concentration of an unchanged form of buprenorphine, improvement in social impairment, and analgesic drug efficacy.
Fig. 6 shows the results of evaluating the sustained improvement effect on sociality of buprenorphine using valproic acid model mice.
Fig. 7 shows the results of evaluating the improvement effect on sociality of buprenorphine using FMR-1 knockout mice.
Fig. 8 shows the results of evaluating the influence of combined use of naloxone on the improvement effect on sociality of buprenorphine using valproic acid model mice.
Fig. 9 shows the results of quantifying activation of Nucleus Accumbens (NAc) by buprenorphine using valproic acid model mice.
Fig. 10 shows the results of quantifying the activation of the medial prefrontal cortex by buprenorphine using valproic acid model mice.
Fig. 11 shows the results of quantifying activation of dorsomedial periaqueductal gray (dmPAG) by buprenorphine using valproic acid model mice.

### [MODE FOR CARRYING OUT THE INVENTION]

The term "consisting of' means having only components. The term "comprising" means being not limited to the components, but not excluding elements that are not described.

Hereinafter, the present invention will be described with reference to embodiments. Throughout the present specification, an expression in a singular form should be understood as also including the concept of its plural form, unless otherwise stated. Therefore, singular articles (for example, "a", "an", "the", and the like in English) should be understood as also including the concept of their plural form, unless otherwise stated.

In addition, the terms used in the present specification should be understood as being used in the meanings commonly used in the art unless otherwise stated. Therefore, unless otherwise defined, all terminology and scientific terms used in the present specification have the same meanings as commonly understood by those skilled in the art to which the present invention belongs. In case of conflict, the present specification (including definitions) prevails.

The "Autism Spectrum Disorder (ASD)" is a type of neurodevelopmental disorder (neurodevelopmental disorder group), and includes autism disorder, Asperger's disorder, and pervasive developmental disorder not otherwise specified. For the term (definition), characteristics of a disorder, and diagnostic criteria, "International Classification of Diseases (ICD) 11th edition" prepared by the World Health Organization (WHO), "DSM-5 (registered trademark) Manual of Diagnosis and Statistics of Mental Diseases" prepared by the American Psychiatric Association, and the like can be referred to. Note that the criteria are not particularly limited to the ICD-11 and the DSM-5 as long as criteria that can be used to accurately diagnose autism spectrum disorder are acceptable.

Core symptoms of autism spectrum disorder include "persistent disorders in social communication and/or social interaction" and "symptoms related to restrictive and repetitive patterns of behaviors, interests, and activities." in patients with autism spectrum disorder, e.g., persistent disorders of social communication and/or social interaction.

The "autism spectrum disorder-like symptom" includes a case in which the diagnostic criteria for autism spectrum disorder are not satisfied, but an autism spectrum disease-like symptom is exhibited. The "autism spectrum disorder-like symptom" further include an autism spectrum disorder-like symptom associated with other diseases (For example, fragile X syndrome, Rett syndrome, tuberous sclerosis (TSC), etc.). The "autism spectrum disorder-like symptoms" include, for example, "disorders in social communication and/or social interaction (including a persistent disorder)" and "symptoms related to restrictive and repetitive patterns of behaviors, interests, and activities.", and are preferably disorders in social communication and/or social interaction (including a persistent disorder).

For the terms (definitions) of "fragile X syndrome" and "Rett syndrome", characteristics of the disorder, and diagnostic criteria, for example, "International Classification of Diseases (ICD) 11th edition" prepared by the World Health Organization (WHO), can be referred to.

As used herein, prevention includes the avoidance of the occurrence of symptoms. Treatment includes alleviation, moderation, amelioration of symptoms.

An "effective amount for treating pain" is a dose effective to treat pain.

As the "effective amount for treating pain", for example, formulations containing 5 mg, 10 mg, and 20 mg are approved as transdermal administration formulations of buprenorphine, and the release rates of buprenorphine in these formulations are 5 µg/h, 10 µg/h, and 20 µg/h, respectively (Non-patent Documents 10 and 11).

As the "effective amount for treating pain", for example, 0.075 mg, 0.15 mg, 0.3 mg, 0.6 mg, 0.75 mg, and 0.9 mg are approved as transmucosal administration formulations of buprenorphine, for example, buccal films. In addition, for example, 200 pg, 400 µg, 2 mg, and 8 mg have been approved as sublingual tablets (Non-patent Documents 12 to 14). For example, 0.2 mg and 0.4 mg have been approved as suppositories (Non-patent Document 15), and 0.2 mg and 0.4 mg have been approved as injections of buprenorphine (Non-patent Document 16).

As the "effective amount for treating pain", for example, 5 mg, 10 mg, 15 mg, 30 mg, and 60 mg are approved as an oral administration formulation of morphine (Non-patent Documents 18 and 21). For example, 10 mg, 50 mg, and 200 mg have been approved as injections of morphine (Non-patent Document 19). For example, 10 mg, 20 mg, and 30 mg have been approved as transmucosal administration formulations (suppositories) of morphine (Non-patent Document 22).

The "minimum effective amount for treating pain" is the minimum dose used for pain treatment. For example, it is the minimum dose approved as a treatment agent for pain.

In one aspect, when buprenorphine is transdermally administered, the minimum effective amount for treating pain includes a formulation comprising about 5 mg. Here, since the transdermal administration formulation of buprenorphine described in Non-patent Documents 7 and 8 is a formulation to be administered once every 7 days, in one aspect, when buprenorphine is transdermally administered, the minimum dose (per day) used for pain treatment is a formulation comprising about 0.7 mg.

In one aspect, when buprenorphine is transdermally administered, the minimum effective amount for treating pain is, for example, about 5 µg/h. The minimum dose (per day) used for pain treatment includes, for example, a dosage amount when administered at about 5 µg/h for 24 hours, that is, about 0.12 mg.

In one aspect, in the case of transmucosal administration (for example, transoral mucosal administration) of buprenorphine, the minimum effective amount for treating pain is about 0.075 mg. In one aspect, about 0.075 mg is a daily dosage amount.

In one aspect, when morphine is orally administered, the minimum effective amount for treating pain is about 5 mg.

In one aspect, when morphine is administered transmucosally or as an injection, the minimum effective amount for treating pain is about 10 mg.

An "effective amount for treating autism spectrum disorder" is an amount effective for treating and/or preventing autism spectrum disorder, fragile X syndrome, and/or the autism spectrum disorder-like symptom. Preferably, it is an amount effective for treating and/or preventing autism spectrum disorder.

In this specification, unless the context dictates otherwise, reciting "about" before a numerical value X includes the range of +/-10% of the numerical value X and includes the numerical value that, having regard to the significant figure of the numerical value X, is rounded to the nearest significant figure.

For example, about 1 ng/mL includes 0.9 ng/mL and 1.4 ng/mL.

The buprenorphine is 21-cyclopropyl-7-α-[(S)-1-hydroxy-1,2,2-trimethylpropyl]-6,14-endo-ethano-6,7,8,14 tetrahydrooripavine.

Buprenorphine is a compound represented by the following formula:

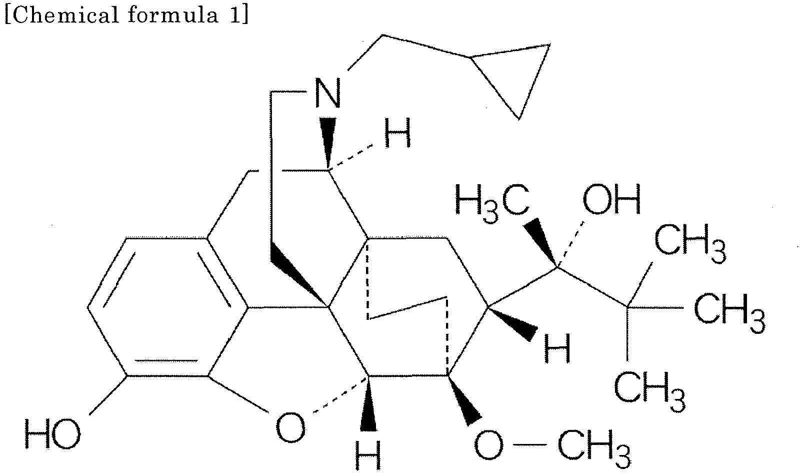

The buprenorphine or the pharmaceutically acceptable salt thereof for use in the present invention may be administered as a possible isomer of buprenorphine (for example, a keto-enol isomer, an imine-enamine isomer, a diastereoisomer, an optical isomer, a rotational isomer, a racemate, or a mixture thereof), a prodrug or a pharmaceutically acceptable salt thereof.

One or more hydrogen atom, carbon atom and/or another atom of the buprenorphine of the present invention may be replaced with an isotope of the hydrogen atom, carbon atom and/or another atom. Examples of such an isotope include hydrogen, carbon, nitrogen, oxygen, such as ²H, ³H, ¹¹C, ¹³C, ¹⁴C, ¹⁵N, ¹⁸O, and ¹⁷O, respectively. The buprenorphine of the present invention also includes all buprenorphine substituted with such an isotope. Buprenorphine substituted with the isomer is also useful as a pharmaceutical product and includes all radiolabeled forms of buprenorphine. Furthermore, a "radiolabeling method" for production of the "radiolabeled form" is also included in the present invention, and the "radiolabeled form" is useful as a tool for metabolic pharmacokinetic studies and for research and/or diagnosis in binding assay.

The radiolabeled form of buprenorphine of the present invention can be prepared by the method well known in the art. For example, a tritium-labeled compound of buprenorphine can be prepared by introducing tritium into buprenorphine by a catalytic dehalogenation reaction using tritium. This method includes reacting an appropriately halogenated precursor of buprenorphine with tritium gas in the presence of an appropriate catalyst, such as Pd/C, and in the presence or absence of a base. The other appropriate method of preparing a tritium-labeled compound can be referred to "Isotopes in the Physical and Biomedical Sciences, Vol. 1, Labeled Compounds (Part A), Chapter 6 (1987)". A ¹⁴C-labeled compound can be prepared by using a raw material having ¹⁴C carbon.

Morphine is a compound represented by the following formula:

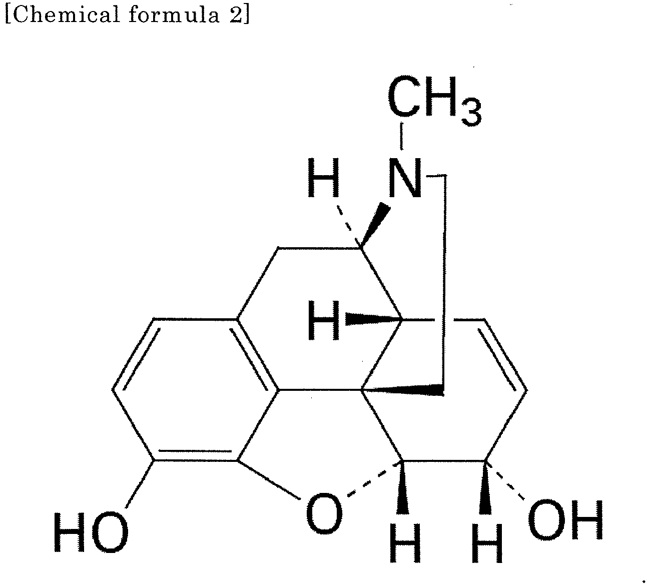

The morphine or the pharmaceutically acceptable salt thereof for use in the present invention may be administered as a possible isomer of morphine (for example, a keto-enol isomer, an imine-enamine isomer, a diastereoisomer, an optical isomer, a rotational isomer, a racemate, or a mixture thereof), a prodrug or a pharmaceutically acceptable salt thereof.

One or more hydrogen, carbon, and/or other atoms in the morphine of the present invention may be substituted with isotopes of hydrogen, carbon, and/or other atoms, respectively. Examples of such an isotope include hydrogen, carbon, nitrogen, oxygen, such as ²H, ³H, ¹¹C, ¹³C, ¹⁴C, ¹⁵N, ¹⁸O, and ¹⁷O, respectively. The morphine of the present invention also includes morphine substituted with such an isotope. Morphine substituted with the isotope is also useful as a pharmaceutical product and includes all radiolabeled forms of morphine. Furthermore, a "radiolabeling method" for production of the "radiolabeled form" is also included in the present invention, and the "radiolabeled form" is useful as a tool for metabolic pharmacokinetic studies and for research and/or diagnosis in binding assay.

The radiolabeled form of morphine of the present invention can be prepared by the method well known in the art. For example, a tritium-labeled compound of morphine can be prepared by introducing tritium into morphine by a catalytic dehalogenation reaction using tritium. This method includes reacting an appropriately halogenated precursor of morphine with tritium gas in the presence of an appropriate catalyst, such as Pd/C, and in the presence or absence of a base. The other appropriate method of preparing a tritium-labeled compound can be referred to "Isotopes in the Physical and Biomedical Sciences, Vol. 1, Labeled Compounds (Part A), Chapter 6 (1987)". A ¹⁴C-labeled compound can be prepared by using a raw material having ¹⁴C carbon.

In the present description, as the "pharmaceutically acceptable salt", examples of basic salts include alkali metal salts such as lithium salt, sodium salt and potassium salt; alkaline earth metal salts such as calcium salt and barium salt; transition metal salts such as zinc salt and iron salt; magnesium salt; ammonium salt; aliphatic amine salts such as trimethylamine salt, triethylamine salt, dicyclohexylamine salt, ethanolamine salt, diethanolamine salt, triethanolamine salt, ethylenediamine salt, meglumine salt and procaine salt; aralkylamine salts such as N,N-dibenzylethylenediamine; aromatic heterocyclic amine salts such as pyridine salt, picoline salt, quinoline salt and isoquinoline salt; quaternary ammonium salts such as tetramethylammonium salt, tetraethylammonium salt, benzyltrimethylammonium salt, benzyltriethylammonium salt, benzyltributylammonium salt, methyltrioctylammonium salt and tetrabutylammonium salt; and basic amino acid salts such as arginine salt and lysine salt. Examples of acidic salts include inorganic acid salts such as hydrochloride, sulfate, nitrate, phosphate, carbonate, hydrogen carbonate, hydrobromate, hydroiodide and perchlorate; organic acid salts such as formate, acetate, propionate, trifluoroacetate, citrate, lactate, tartrate, oxalate, maleate, fumarate, succinate, mandelate, glutarate, malate, benzoate, phthalate and ascorbate; sulfonates such as methanesulfonate, ethanesulfonate, isethionate, benzenesulfonate and p-toluenesulfonate; and acidic amino acid salts such as aspartate and glutamate. These salts can be formed by a conventional method.

As the "pharmaceutically acceptable salt", an acidic salt is preferable, a hydrochloride or a sulfate is more preferable, and a hydrochloride is particularly preferable.

The buprenorphine or the pharmaceutically acceptable salt thereof and morphine or pharmaceutically acceptable salt thereof used in the present invention may be solvates, co-crystals and/or crystal polymorphs thereof.

Solvates include organic solvates in which any number of organic solvent molecules are coordinated and hydrates in which any number of water molecules are coordinated. In the present description, the term "solvate" means a solvate of the compound or a pharmaceutically acceptable salt thereof, and examples thereof include a monosolvate, a disolvate, a monohydrate and a dihydrate. Examples thereof include a hydrate, an ethanol hydrate, a methyl acetate hydrate, an ethyl acetate and a 2-propanol hydrate, an n-propyl acetate and a 2-propanol hydrate, an acetonitrile hydrate, a 1,2-dimethoxyethane hydrate, and a methyl isobutyl ketone hydrate, and preferably include a hydrate such as a monohydrate, for example, trihydrate.

One aspect of buprenorphine or the pharmaceutically acceptable salt thereof includes buprenorphine hydrochloride.

One aspect of morphine or pharmaceutically acceptable salt thereof includes morphine hydrochloride (for example, morphine hydrochloride hydrate) and morphine sulfate.

A pharmaceutical composition can be obtained by mixing an effective amount of the compound used in the present invention with various pharmaceutical additives appropriate for the dosage form, such as an excipient, a binder, a disintegrating agent, and a lubricating agent, as necessary. Furthermore, the pharmaceutical composition can be prepared into a pharmaceutical composition for use for a child, an elderly, a patient with a serious case, or a surgical operation, by appropriately changing the effective amount of the compound, the dosage form, and/or various pharmaceutical additives.

The administration route of the pharmaceutical composition of the present invention is not particularly limited, and the pharmaceutical composition can be administered orally or parenterally. Examples of a method of parenteral administration include transdermal, subcutaneous, intravenous, intraarterial, intramuscular, intraperitoneal, transmucosal, inhalation, transnasal, ophthalmic, inner ear, or vaginal administration.

As one aspect, the administration route of the pharmaceutical composition of the present invention is transdermal or transmucosal administration (for example, transoral mucosa, sublingual, a suppository, preferably transoral mucosa, sublingual), for example, transdermal administration.

In the case of oral administration, the pharmaceutical composition may be prepared into any dosage form that is commonly used, such as a solid formulation for internal use (e.g., a tablet, a powder, a granule, a capsule, a pill, a film, or the like) or a liquid formulation for internal use (e.g., a suspension, an emulsion, an elixir, a syrup, a lemonade, a spirit, an aromatic water, an extract, a decoction, a tincture, or the like), and administered. The tablet may be a sugar-coated tablet, a film-coated tablet, an enteric-coated tablet, a sustained-release tablet, a troche tablet, a sublingual tablet, a buccal tablet, a chewable tablet, or an orally disintegrating tablet; the powder and the granule may be a dry syrup; and the capsule may be a soft capsule, a micro capsule, or a sustained-release capsule.

In the case of parenteral administration, the pharmaceutical composition can be suitably administered in any dosage form that is commonly used, such as an injection, an infusion, or a preparation for external use (e.g., an eye drop, a nasal drop, an ear drop, an aerosol, an inhalant, a lotion, an impregnating agent, a liniment, a gargling agent, an enema, an ointment, a plaster, a jelly, a cream, a patch, a poultice, a powder for external use, a suppository, or the like). The injection may be an emulsion of O/W, W/O, O/W/O, W/O/W type, or the like.

The pharmaceutical composition for transdermal administration is not particularly limited, and examples thereof include an external solid agent (for example, external powders), an external liquid agent (for example, a liniment agent and a lotion agent), spray (an external aerosol, pump spray), ointment, cream, gel, and a patch (for example, a tape agent (for example, a plaster agent, plaster) or a cataplasm agent). For example, the patch is used.

An aspect of the method, the agent, and the pharmaceutical composition of the present invention is not particularly limited, and can be appropriately determined according to the degree of a symptom, the body weight, the age, the administration form of the agent, and the like of the patient. Aspects of the present invention are exemplified below. As aspects of the method, pharmaceutical preparation, and pharmaceutical composition of the present invention, aspects and ranges of all possible combinations of the above items and the items exemplified below are exemplified.

Buprenorphine or the pharmaceutically acceptable salt thereof and/or morphine or the pharmaceutically acceptable salt thereof are administered at a dose less than or equal to an effective amount for treating pain (for example, a dose less than or equal to about 1/2 times, less than or equal to about 1/10 times of an effective amount for treating pain).

Buprenorphine or the pharmaceutically acceptable salt thereof and/or morphine or the pharmaceutically acceptable salt thereof is administered at a dose of less than or equal to about 3 times (for example, less than about 3 times), less than or equal to about 2 times (for example, less than or equal to about 1 times, less than or equal to about 1/2 times, less than or equal to about 1/3 times, or about less than or equal to about 1/4 times) of a minimum effective amount for treating pain.

Buprenorphine or the pharmaceutically acceptable salt thereof and/or morphine or the pharmaceutically acceptable salt thereof is administered at equal to or more than about 1/33 times (for example, a dosage amount of more than about 1/33 times) the minimum effective amount for treating pain, and equal to or more than about 1/30 times (for example, equal to or more than about 1/25 times, equal to or more than about 1/20 times, equal to or more than about 1/10 times) the minimum effective amount for treating pain.

The buprenorphine or the pharmaceutically acceptable salt thereof and/or morphine or the pharmaceutically acceptable salt thereof is administered at about 1/33 to about 3 times (for example, more than about 1/33 times and less than about 3 times), about 1/30 to about 3 times (for example, equal to or more than about 1/30 times and less than about 3 times), about 1/30 to about 2 times, about 1/30 to about 1 times, about 1/30 to about 1/2 times, about 1/20 to about 3 times (for example, equal to or more than about 1/20 times and less than about 3 times), about 1/20 to about 2 times, about 1/20 to about 1 times, about 1/30 to about 1/2 times, about 1/10 to about 3 times (for example, equal to or more than about 1/10 times and less than about 3 times), about 1/10 to about 2 times, about 1/10 to about 1 times, about 1/10 to about 1/2 times the minimum effective amount for treating pain.

The plasma concentration (preferably the maximum plasma concentration) of buprenorphine or the pharmaceutically acceptable salt thereof is, for example, about 2 ng/mL or less, about 1.5 ng/mL or less, about 1 ng/mL or less, about 0.9 ng/mL or less, about 0.8 ng/mL or less, or about 0.7 ng/mL or less. The concentration is preferably about 0.6 ng/mL or less, about 0.62 ng/mL or less, less than about 0.6 ng/mL, less than about 0.62 ng/mL, about 0.5 ng/mL or less, about 0.4 ng/mL or less, or about 0.3 ng/mL or less. More preferably, it is about 0.2 ng/mL or less, for example, about 0.18 ng/mL or less, about 0.17 ng/mL or less, about 0.15 ng/mL or less, about 0.1 ng/mL or less, about 90 pg/mL or less, about 80 pg/mL or less, about 70 pg/mL or less, about 60 pg/mL or less, or about 50 pg/mL or less (The plasma concentration is expressed as a concentration in terms of buprenorphine without considering the salt moiety. The same applies hereinafter).

The plasma concentration (preferably the maximum plasma concentration) of buprenorphine or the pharmaceutically acceptable salt thereof is, for example, a concentration of about 0.1 pg/mL or more, about 0.5 pg/mL or more, about 1 pg/mL or more, about 2 pg/mL or more, about 3 pg/mL or more, more than about 3 pg/mL, or more than about 3.3 pg/mL. Preferably, the concentration is, for example, about 4 pg/mL or more, more than about 4 pg/mL, about 5 pg/mL or more, about 6 pg/mL or more, about 7 pg/mL or more, about 8 pg/mL or more, about 9 pg/mL or more, about 10 pg/mL or more, about 11 pg/mL or more, about 12 pg/mL or more, about 13 pg/mL or more, about 14 pg/mL or more, about 15 pg/mL or more, about 16 pg/mL or more, about 17 pg/mL or more, or about 18 pg/mL or more.

The concentration is, for example, about 0.9 ng/mL or less, about 0.8 ng/mL or less, or about 0.7 ng/mL or less. Preferably, the concentration is, for example, about 0.6 ng/mL or less, about 0.62 ng/mL or less, less than about 0.6 ng/mL, less than about 0.62 ng/mL, about 0.5 ng/mL or less, about 0.4 ng/mL or less, or about 0.3 ng/mL or less. More preferably, the concentration is, for example, about 0.2 ng/mL or less, about 0.18 ng/mL or less, about 0.17 ng/mL or less, about 0.15 ng/mL or less, about 0.1 ng/mL or less, about 90 pg/mL or less, about 80 pg/mL or less, about 70 pg/mL or less, about 60 pg/mL or less, about 50 pg/mL or less, or about 40 pg/mL or less.

The plasma concentration (preferably the maximum plasma concentration) of buprenorphine or the pharmaceutically acceptable salt thereof is, for example,
about 1 pg/mL to about 2 ng/mL or less, about 1 pg/mL to about 1 ng/mL or less, about 1 pg/mL to about 0.6 ng/mL, about 1 pg/mL to about 0.2 ng/mL, about 1 pg/mL to about 0.1 ng/mL, about 1 pg/mL to about 50 pg/mL,
about 3 pg/mL to about 2 ng/mL or less, about 3 pg/mL to about 1 ng/mL or less, about 3 pg/mL to about 0.6 ng/mL, about 3 pg/mL to about 0.3 ng/mL, about 3 pg/mL to about 0.2 ng/mL, about 3 pg/mL to about 0.1 ng/mL, about 3 pg/mL to about 50 pg/mL,
about 4 pg/mL to about 2 ng/mL or less, about 4 pg/mL to about 1 ng/mL or less, about 4 pg/mL to about 0.6 ng/mL, about 4 pg/mL to about 0.2 ng/mL, about 4 pg/mL to about 0.1 ng/mL, about 4 pg/mL to about 50 pg/mL,
about 5 pg/mL to about 2 ng/mL or less, about 5 pg/mL to about 1 ng/mL or less, about 5 pg/mL to about 0.6 ng/mL, about 5 pg/mL to about 0.2 ng/mL, about 5 pg/mL to about 0.1 ng/mL, about 5 pg/mL to about 50 pg/mL,
about 6 pg/mL to about 2 ng/mL or less, about 6 pg/mL to about 1 ng/mL or less, about 6 pg/mL to about 0.6 ng/mL, about 6 pg/mL to about 0.2 ng/mL, about 6 pg/mL to about 0.1 ng/mL, about 5 pg/mL to about 50 pg/mL,
about 8 pg/mL to about 2 ng/mL or less, about 8 pg/mL to about 1 ng/mL or less, about 8 pg/mL to about 0.6 ng/mL, about 8 pg/mL to about 0.2 ng/mL, about 8 pg/mL to about 0.1 ng/mL, about 8 pg/mL to about 80 pg/mL, about 8 pg/mL to about 50 pg/mL,
about 10 pg/mL to about 2 ng/mL or less, about 10 pg/mL to about 1 ng/mL or less, about 10 pg/mL to about 0.6 ng/mL, about 10 pg/mL to about 0.2 ng/mL, about 10 pg/mL to about 0.1 ng/mL, about 10 pg/mL to about 50 pg/mL,
about 15 pg/mL to about 2 ng/mL or less, about 15 pg/mL to about 1 ng/mL or less, about 15 pg/mL to about 0.6 ng/mL, about 15 pg/mL to about 0.2 ng/mL, about 15 pg/mL to about 0.1 ng/mL, about 15 pg/mL to about 50 pg/mL,
about 18 pg/mL to about 2 ng/mL or less, about 18 pg/mL to about 1 ng/mL or less, about 18 pg/mL to about 0.6 ng/mL, about 18 pg/mL to about 0.2 ng/mL, about 18 pg/mL to about 0.17 ng/mL, about 18 pg/mL to about 0.1 ng/mL, about 18 pg/mL to about 50 pg/mL,
about 20 pg/mL to about 2 ng/mL or less, about 20 pg/mL to about 1 ng/mL or less, about 20 pg/mL to about 0.6 ng/mL, about 20 pg/mL to about 0.2 ng/mL, about 20 pg/mL to about 0.1 ng/mL, or about 20 pg/mL to about 50 pg/mL.

As a further aspect, for any of the plasma concentration ranges described above, a range not including numerical values at both ends (for example, for about 3 pg/mL to about 0.6 ng/mL, more than about 3 pg/mL and less than about 0.6 ng/mL) is exemplified.

As one aspect, the plasma concentration is the maximum plasma concentration in a steady state.

As one aspect, the plasma concentration is a plasma concentration in a steady state.

As one aspect, the plasma concentration is continuously maintained for 30 minutes or more, for example, 1 hour or more, for example, 2 hours or more.

The content or dosage amount (for example, the content or dosage amount in the pharmaceutical composition for transdermal administration) of buprenorphine or the pharmaceutically acceptable salt thereof is, for example, about 20 mg or less, about 10 mg or less, about 7 mg or less, about 5 mg or less, about 4 mg or less, about 2.5 mg or less, about 2 mg or less, about 1 mg or less, about 0.7 mg or less, about 0.5 mg or less, about 0.4 mg or less, about 0.3 mg or less, about 0.2 mg or less, or about 0.1 mg or less (The content or dosage amount is expressed in terms of a buprenorphine amount without considering a salt moiety. The same applies hereinafter).

The content or dosage amount (for example, the content or dosage amount in the pharmaceutical composition for transdermal administration) of buprenorphine or the pharmaceutically acceptable salt thereof is, for example, about 0.001 mg or more, about 0.003 mg or more, about 0.005 mg or more, about 0.01 mg or more, about 0.03 mg or more, about 0.05 mg or more, about 0.1 mg or more, about 0.3 mg or more, or about 0.5 mg or more.

The content of buprenorphine or the pharmaceutically acceptable salt thereof of the present invention in a pharmaceutical composition for transdermal administration may be about 0.56 mg to about 1.7 mg, or may be about 0.56 mg or about 1.7 mg.

The content or dosage amount (for example, the dosage amount or release rate in a pharmaceutical composition for transdermal administration) of buprenorphine or the pharmaceutically acceptable salt thereof is, for example, about 20 µg/h or less, about 10 µg/h or less, about 5 µg/h or less, about 2.5 µg/h or less, about 3 µg/h or less, about 2 µg/h or less, about 1 µg/h or less, about 0.1 µg/h or less (The release rate is expressed in terms of a buprenorphine amount without considering a salt moiety. The same applies hereinafter).

The content or dosage amount (for example, the dosage amount or release rate in a pharmaceutical composition for transdermal administration) of buprenorphine or the pharmaceutically acceptable salt thereof is, for example, about 0.01 µg/h or more, about 0.05 µg/h or more, about 0.1 µg/h or more, about 0.5 µg/h or more, or about 1 µg/h or more.

The release rate of the pharmaceutical composition for transdermal administration of buprenorphine or the pharmaceutically acceptable salt thereof of the present invention may be about 0.56 µg/h to about 1.7 µg/h, and may be about 0.56 µg/h or about 1.7 µg/h.

The content or dosage amount (for example, the content or dosage amount in a pharmaceutical composition for transmucosal administration) of buprenorphine or the pharmaceutically acceptable salt thereof is, for example, about 1 mg or less, about 0.5 mg or less, about 0.2 mg or less, about 0.1 mg or less, about 0.075 mg or less, about 0.05 mg or less, or about 0.01 mg or less.

The content or dosage amount (for example, the content or dosage amount in a pharmaceutical composition for transmucosal administration) of buprenorphine or the pharmaceutically acceptable salt thereof is, for example, about 0.00001 mg or more, about 0.0001 mg or more, about 0.001 mg or more, or about 0.01 mg or more.

The content or dosage amount of buprenorphine or the pharmaceutically acceptable salt thereof is, for example, about 0.3 µg/kg to about 30 pg/kg(For example, more than about 0.3 µg/kg and less than about 30 µg/kg), about 0.5 µg/kg to about 20 pg/kg, about 1 µg/kg to about 10 µg/kg.

For example, when administered to a 60 kg patient, it is, for example, about 18 µg to about 1.8 mg (for example, more than about 18 µg and less than about 1.8 mg), about 30 µg to about 1.2 mg, about 60 µg to about 0.6 mg.

For example, when administered to a 40 kg patient, it is, for example, about 12 µg to about 1.2 mg (for example, more than about 12 µg and less than about 1.2 mg), about 20 µg to about 0.8 mg, about 40 µg to about 0.4 mg.

For example, when administered to a 20 kg patient, it is, for example, about 6 µg to about 0.6 mg (for example, more than about 6 µg and less than about 0.6 mg), about 10 µg to about 0.4 mg, about 20 µg to about 0.2 mg.

The plasma concentration (preferably the maximum plasma concentration) of morphine or the pharmaceutically acceptable salt thereof is, for example, about 500 ng/mL or less, about 350 ng/mL or less, about 300 ng/mL or less, about 200 ng/mL or less, about 100 ng/mL or less, about 10 ng/mL or less, or about 7 ng/mL or less.

The plasma concentration (preferably the maximum plasma concentration) of morphine or the pharmaceutically acceptable salt thereof is, for example, about 0.1 ng/mL or more, about 0.5 ng/mL or more, about 1 ng/mL or more, about 2 ng/mL or more, about 5 ng/mL or more, or about 10 ng/mL or more.

As one aspect, the plasma concentration is the maximum plasma concentration in a steady state.

As one aspect, the plasma concentration is a plasma concentration in a steady state.

As one aspect, the plasma concentration is continuously maintained for 30 minutes or more (for example, 1 hour or more, 2 hours or more).

The content or dosage amount of morphine or pharmaceutically acceptable salt thereof is, for example, 10 mg or less, 5 mg or less, 3 mg or less, 1 mg or less, or 0.1 mg or less.

The content or dosage amount of morphine or pharmaceutically acceptable salt thereof is, for example, 0.01 mg or more, 0.05 mg or more, 0.1 mg or more, 0.5 mg or more, or 1 mg or more.

The content or dosage amount of morphine or pharmaceutically acceptable salt thereof is, for example, about 0.03 mg/kg to about 0.3 mg/kg (for example, more than about 0.03 mg/kg and less than about 0.3 mg/kg), about 0.03 mg/kg to about 0.1 mg/kg.

For example, when administered to a 60 kg patient, it is, for example, about 1.8 mg to about 18 mg (for example, more than about 1.8 mg and less than about 18 mg), about 1.8 mg to about 6 mg.

For example, when administered to a 40 kg patient, it is, for example, about 1.2 mg to about 12 mg (for example, more than about 1.2 mg and less than about 12 mg), about 1.2 mg to about 4 mg.

For example, when administered to a 20 kg patient, it is, for example, about 0.6 mg to about 6 mg (for example, more than about 0.6 mg and less than about 6 mg), about 0.6 mg to about 2 mg.

As one aspect, the method, agent, pharmaceutical preparation, and pharmaceutical composition of the present invention may contain a single active ingredient and be administered alone.

In one aspect, the administration interval is about 12 hours or more (for example, about 24 hours, about 24 hours or more).

As one aspect, the number of doses is twice or less (for example, once per day) per day.

The method, agent, pharmaceutical preparation, and pharmaceutical composition, etc. of the present invention can include attachment and label which are described for providing instructions to paramedical personnel undertaking prevention or treatment such as physicians, for specification of a patient or a subject targeted by the present invention as a therapeutic objective, and guidelines for treatment and/or prevention such as dosage and administration, and precautions. However, these public documents are not limited to paper media, but can be provided through the Internet, and guidelines for prevention or treatment can be provided to physicians and the like on the basis of various other information sources in addition to public documents. Therefore, it should be understood that the present invention also includes embodiments that are used on the basis of information other than attachment and label.

As aspects of the method, agent, pharmaceutical preparation, and pharmaceutical composition of the present invention, they may be used in combination with other psychiatric drugs (including digital drugs) and/or other therapies (including various treatment methods without a drug), or may be used without these.

The pharmaceutical composition or method of the present invention preferably has one or more superior characteristics selected from the following.
a) It is useful in the treatment and/or prevention of various psychiatric diseases, in particular autism spectrum disorder, fragile X syndrome, the autism spectrum disorder-like symptom, and/or disorders in social communication and/or social interaction. Preferably, it is useful for treatment and/or prevention of autism spectrum disorder. Particularly preferably, it is useful for the treatment and/or prevention of a core symptom of autism spectrum disorder.
b) The effective drug efficacy amount and concentration are low. Therefore, a pharmaceutical product having a high safety margin can be obtained. In addition, it may be a pharmaceutical product having a low risk of a side action.
c) Drug efficacy exhibits at a dose (or plasma concentration) equal to or preferably lower than the approved dose as a treatment agent for pain. Therefore, safety as a pharmaceutical product is high. In addition, for example, there is a possibility that the restriction as a controlled drug is small. For example, no special program (REMS or the like) is required for administration.
d) Duration of drug efficacy is long. As a result, treatment with a small number of doses is possible, and for example, it is expected that the drug-taking compliance of the patient is high. Furthermore, for example, in a case where the patient is a child, it is expected that the burden on the caregiver is reduced.

Buprenorphine and the pharmaceutically acceptable salt thereof and morphine or the pharmaceutically acceptable salt thereof can be synthesized according to the known method.

### [EXAMPLES]

Hereinafter, the present invention will be described based on examples. However, the present invention is not limited to these examples and the like.

### (Example 1: Evaluation of drug efficacy by valproic acid model mice)

The valproic acid model mice (VPA model) are ASD model mice based on clinical evidence that, in humans, taking valproic acid during pregnancy significantly increases the probability that a neonate will suffer from ASD. Since ASD-like symptoms such as social impairment and cognitive dysfunction are observed, it is one of the most widely used the ASD model mice.

In a VPA model produced by administering valproic acid to ICR mice in fetal period as described in previous studies (Hara Y, Ago Y, Higuchi M, Hasebe S, Nakazawa T, Hashimoto H, Matsuda T, Takuma K. Oxytocin attenuates deficits in social interaction but not recognition memory in a prenatal valproic acid-induced mouse model of autism. Horm Behav. 2017 Nov; 96: 130-136. doi: 10.1016/j.yhbeh.2017.09.013. Epub 2017 Sep 28. PMID: 28942000), MOR agonists morphine, buprenorphine, or tramadol, which are used as an analgesic, was administered once, and the action of the VPA model on social impairment was examined.

As the sociality evaluation, a method called a social interaction test was used. In the Social Interaction Test, two mice are placed in one cage, and how many seconds a target mouse (Test mouse) performs sniffing, which is social behavior, on another mouse (Intruder mouse) is quantified. Saline, morphine hydrochloride, buprenorphine hydrochloride, or tramadol hydrochloride was subcutaneously administered, and the amount of social behavior for 20 minutes from 1 hour after administration was quantified. The doses were calculated as hydrochloride for morphine and tramadol and as free form for buprenorphine.

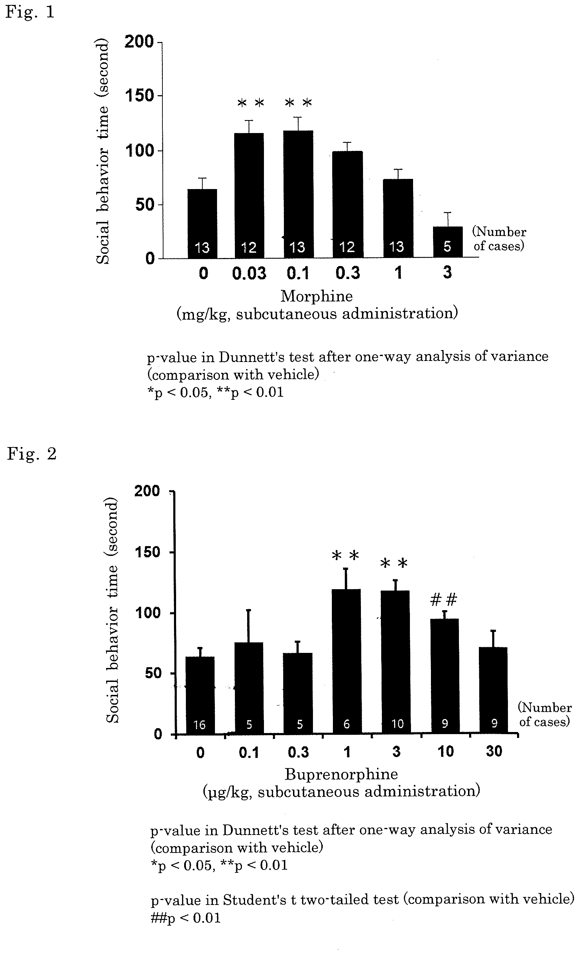

The results are shown in Figs. 1 to 3.

For morphine and buprenorphine, a significant improvement effect in social impairment was observed at a low dose of less than 1/10 of the dose (8 mg/kg, 1 mg/kg, Non-patent Document 28) at which the respective analgesic effects of morphine and buprenorphine were maximum. On the other hand, at a high dose close to the analgesic dose, characteristic dose dependence was observed in which both compounds had a reduced the improvement effect in social impairment.

In addition, tramadol, which is used as an analgesic similarly to morphine and buprenorphine and has MOR agonist activity in vivo, was not observed to have the improvement effect in social impairment.

From the above results, morphine and buprenorphine have an improvement effect in social impairment in ASD, and are considered to be useful as a treatment agent for ASD. In addition, although morphine and buprenorphine have enhanced an analgesic effect in the high dose range, it has been surprisingly found that morphine and buprenorphine have a tendency to exhibit high drug efficacy particularly in the low dose range in the improvement effect on sociality.

In particular, since buprenorphine improved the social impairment at a lower dose than morphine, it is expected as a highly safe ASD treatment agent with a low risk of a side action.

### (Example 2: Pharmacokinetic)

To ICR mice, morphine hydrochloride or buprenorphine was adjusted to each dose with Saline and subcutaneously administered, blood collection and brain extraction were performed at each time point after administration, and drug concentrations in plasma and brain were measured by LC-MS. The dose was calculated as a hydrochloride for morphine and as a free form for buprenorphine. The results are shown in Tables 1 and 2.

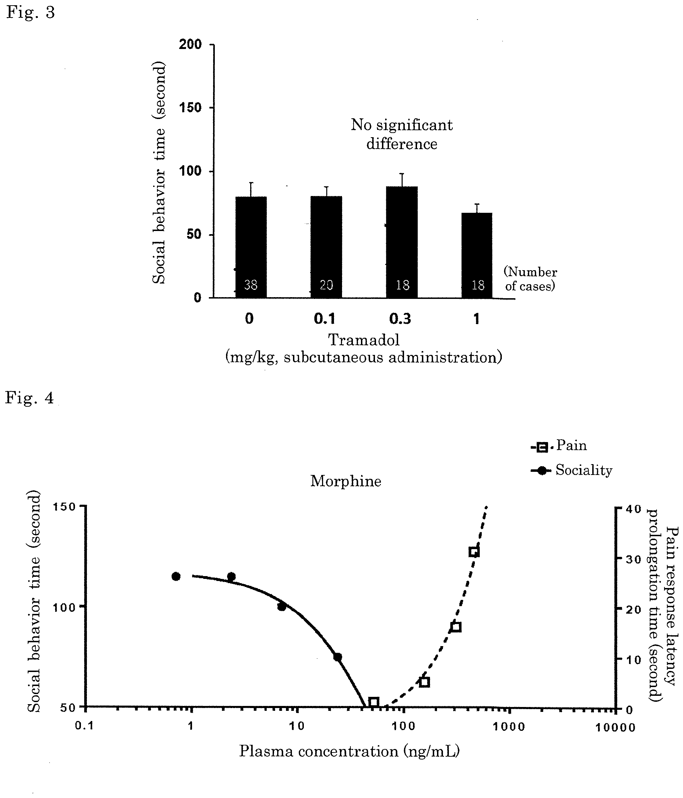

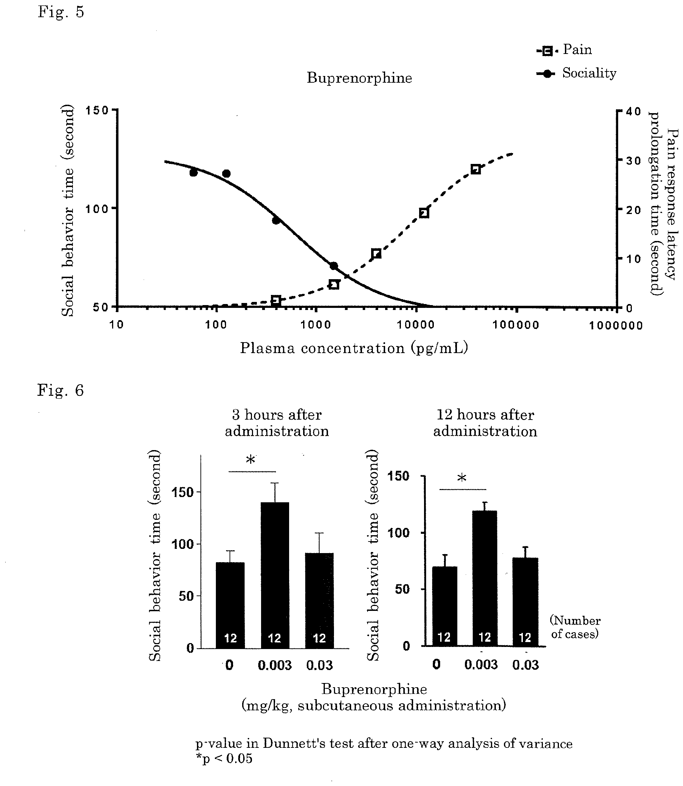

Fig. 4 and Fig. 5 show the relationship between the plasma concentrations of unchanged forms of morphine and buprenorphine, improvement in social impairment, and analgesic drug efficacy.

The left axes of Figs. 4 and 5 indicate the improvement effect in social impairment measured in the same manner as in Example 1, and the right axis indicates the analgesic effect, and the data of Non-patent Document 28 Fig. 4 is cited, and the relationship between the plasma concentrations of unchanged forms of morphine and buprenorphine, the social impairment improvement, and the analgesic drug efficacy is graphed. The horizontal axis represents the concentration of the unchanged form in the plasma calculated from the administration dose at the time of evaluating drug efficacy. With respect to both morphine and buprenorphine, the plasma drug concentration at which the improvement effect in social impairment is observed is lower than the concentration at which the analgesic effect is observed, and is deviated by 10 times or more from the concentration at which maximum drug efficacy is exhibited.

Table 1 shows the time course of the plasma concentrations of the unchanged form of morphine and M6G metabolites.

**[Table 1]**

| Plasma concentration | | | Morphine (ng/mL) | | | | M6G [ng/mL] | | |
|---|---|---|---|---|---|---|---|---|---|
| | | After 15 minutes | After 30 minutes | After 60 minutes | After 90 minutes | After 15 minutes | After 30 minutes | After 60 minutes | After 90 minutes |
| 0.03mg/kg Administration | N=1 | 3.36 | 1.39 | 0.606 | 0 | 10.4 | 6.58 | 6.28 | 0 |
| | N=2 | 1.74 | 0.993 | 0 | 0 | 15.7 | 11.3 | 5.05 | 0.806 |
| | N=3 | 1.9 | 0.966 | 0 | 0 | 13.6 | 10.8 | 3.95 | 0.657 |
| | Average value | 2.33 | 1.12 | 0.202 | 0 | 13.2 | 9.56 | 5.09 | 0.488 |
| 0.1mg/kg Administration | N=1 | 7.65 | 3.61 | 0 | 0 | 47.7 | 63.3 | 0 | 2.98 |
| | N=2 | 5.62 | 2.99 | 0.579 | 0 | 35.3 | 31.7 | 15.3 | 4.4 |
| | N=3 | 8.85 | 2.47 | - * | 0 | 50.7 | 28.5 | - * | 2.85 |
| | Average value | 7.37 | 3.02 | 0.29 | 0 | 44.6 | 41.2 | 7.65 | 3.41 |
| 3mg/kg Administration | N=1 | 284 | 241 | 71 | 16 | 1250 | 906 | 829 | 320 |
| | N=2 | 442 | 123 | 74.1 | 16.9 | 1340 | 752 | 580 | 240 |
| | N=3 | 361 | 105 | 70 | 32.2 | 998 | 822 | 528 | 335 |
| | Average value | 362 | 156 | 71.7 | 21.7 | 1196 | 827 | 646 | 298 |

| | | | | | | | | | |
|---|---|---|---|---|---|---|---|---|---|
| * Data (morphine: 23.8 ng/mL, M6G: 321 ng/mL) of N=3 after 60 minutes at the time of administration of 0.1 mg/kg on was excluded as an outlier and calculated since it was considered to be an obvious measurement error. | | | | | | | | | |

Table 2 shows the time course of the plasma concentration of the unchanged form of buprenorphine.

**[Table 2]**

| Dosage amount (µg/kg) | Sample No. | Plasma concentration (pg/mL) | | | | | Cₘₐₓ (pg/mL) |
|---|---|---|---|---|---|---|---|
| | | 0.5 hr | 1 hr | 3 hr | 12 hr | 24 hr | |
| 0.1 | 1 | 9.98 | 4.52 | BLQ | BLQ | BLQ | |
| | 2 | 6.85 | 4.66 | 2.99 | BLQ | BLQ | |
| | 3 | 6.52 | 5.36 | BLQ | BLQ | BLQ | |
| | Mean | 7.78 | 4.85 | 1 | 0 | 0 | 7.78 |
| | ±SD | 1.91 | 0.45 | 1.73 | | | |
| 0.3 | 1 | 22.8 | 14.3 | 4.36 | BLQ | BLQ | |
| | 2 | 26 | 13.7 | 5.08 | BLQ | BLQ | |
| | 3 | 30.9 | 16.5 | 4.87 | BLQ | BLQ | |
| | Mean | 26.6 | 14.8 | 4.77 | 0 | 0 | 26.6 |
| | ±SD | 4.1 | 1.5 | 0.37 | | | |
| 1 | Measured value | *67* | *25* | *20* | - | - | *67* |
| 3 | 1 | 274 | 132 | 61.3 | 2.31 | BLQ | |
| | 2 | 260 | 119 | 47.6 | 3.58 | BLQ | |
| | 3 | 271 | 127 | 83.3 | 3.46 | BLQ | |
| | Mean | 268 | 126 | 64.1 | 3.12 | 0 | 268 |
| | ±SD | 7 | 7 | 18 | 0.7 | | |
| 10 | 1 | 632* | 358* | 160 | 17.1 | BLQ | |
| | 2 | 711* | 471* | 145 | 27.1 | 2.33 | |
| | 3 | 666* | 367* | 135 | 6.69 | BLQ | |
| | Mean | 670 | 399. | 147 | 17 | 0.777 | 670 |
| | ±SD | 40 | 63 | 13 | 10.2 | 1.35 | |
| 30 | 1 | 1760* | 1320* | 412* | 31.2 | 4.64 | |
| | 2 | 2560* | 1480* | 436* | 72.7 | 7.68 | |
| | 3 | 2770* | 1670* | 494* | 31.3 | 4.2 | |
| | Mean | 2360 | 1490 | 447 | 45.1 | 5.51 | 2360 |
| | ±SD | 530 | 180 | 42 | 23.9 | 1.89 | |

| | | | | | | | |
|---|---|---|---|---|---|---|---|
| BLQ: LLOQ (lower limit of quantitation: 2 pg/mL) or less *: Samples were quantified after 10 fold dilution. | | | | | | | |

### (Example 3: Confirmation of continuous effect)

Saline or buprenorphine was subcutaneously administered to the VPA model in the same manner as in Example 1, and the amount of social behavior for 20 minutes from 3 hour after administration or for 20 minutes from 12 hours after administration was quantified. The results are shown in Fig. 6. The improvement effect in social impairment by administration of 0.003 mg/kg of buprenorphine was observed even 3 hours and 12 hours after administration.

On the other hand, it has been reported that oxytocin under development as a treatment agent for autism spectrum disorder exhibits an improvement effect of social impairment in a model similar to this experiment, but the drug efficacy disappears 3 hours after administration (Horm Behav. 2017 Nov; 96: 130-136).

Therefore, it was suggested that buprenorphine has higher persistence of drug efficacy in improvement in social impairment than oxytocin, and can be a useful treatment agent.

As shown in Table 2, in each of 0.003 mg/kg administration and 0.03 mg/kg administration, the buprenorphine plasma concentration at 12 hours after administration was lower than the plasma concentration at 3 hours after administration, but in Fig. 6, there was no clear difference in the tendency of the improvement effect on sociality at 3 hours after administration and 12 hours after administration. This suggests that, in particular, the maximum plasma concentration (Cmax) affects the improvement effect on sociality.

### (Example 4: Estimation of preferred buprenorphine dosage amount in human)

From Figs. 2 and 3, buprenorphine showed a significant improvement effect on sociality at 1 µg/kg to 10 µg/kg in VPA model mice.

From Example 2, Cmax of buprenorphine at the time of administration of 0.3 µg/kg and at the time of administration of 30 µg/kg were 26.6 pg/mL and 2360 pg/mL, respectively. Thus, a preferred maximum plasma concentration in ASD applications in mice is in the range of about 26.6 pg/mL to about 2360 pg/mL (preferably more than about 26.6 pg/mL and less than about 2360 pg/mL). Here, Cmax of buprenorphine at the time of administration of 1 µg/kg and at the time of administration of 10 µg/kg, which showed a significant improvement effect on sociality, are 67 pg/mL (estimated) and 670 pg/mL, respectively, and thus the particularly preferable range of the maximum plasma concentration in mice includes about 67 pg/mL to 670 pg/mL.

Here, in mice, in the evaluation of analgesic effect, a latent response is observed at doses of 10 µg/kg or more subcutaneous administration (Non-patent Document 28), and thus 10 µg/kg is the minimum dose at which the analgesic effect is observed.

Here, according to Butrans (trademark) package insert, which is a transdermal administration formulation, Cmax at an approved minimum dose (5 mg formulation, release rate 5 µg/h) is 176 pg/mL. According to BELBUCA (registered trademark) package insert, which is a transoral mucosal administration formulation, Cmax at an approved minimum dose (75 pg) is 0.17 ± 0.30 ng/mL. According to the attachment of NORSPAN TAPE (registered trademark), which is a transdermal administration formulation, Cmax at the approved minimum dose (5 mg formulation, release rate 5 µg/h) is 84 µg ± 19/mL. Therefore, administration of buprenorphine at 10 µg/kg (Cmax: 670 pg/mL) in mice is considered to result in a Cmax of buprenorphine in a human of about 84 pg/mL to 176 pg/mL.

As described above, preferred maximum plasma concentrations in ASD applications in mice are in the range of 26.6 pg/mL (at the time of administration of 0.3 µg/kg) to 2360 pg/mL (at the time of administration of 30µ/kg) (preferably more than about 26.6 pg/mL and less than about 2360 pg/mL). By proportional calculation, the maximum plasma concentration preferred in ASD applications in humans was estimated to be in the range of about 3.3 pg/mL to about 620 pg/mL (preferably more than about 3.3 pg/mL and less than about 620 pg/mL). Furthermore, the particularly preferred maximum plasma concentration in humans corresponding to the particularly preferred range of the maximum plasma concentration in mice (67 pg/mL to 670 pg/mL) was estimated to be about 8.4 pg/mL to about 176 pg/mL.

In addition, in mice, a significant improvement effect on sociality was observed at the time of administration of 10 pg/kg, which is the minimum dose at which the analgesic effect is observed, and at the time of administration of 1 pg/kg, which is the 1/10 amount thereof. Stated another way, a particularly preferred dosage amount of buprenorphine thus includes a dosage amount of about 1/10 to about 1 times of the minimum dose approved as a treatment agent for pain (for example, a transdermal administration formulation comprising 5 mg (formulation for administration for 7 days, with a daily dose of about 0.7 mg), a transdermal administration formulation having a release rate of 5 µg/h, a 75 µg transoral mucosal administration formulation, and the like.). In addition, the dosage amounts corresponding to the time of administration 0.3 µg/kg and the time of administration of 30 µg/kg in mice are about 1/33 times and about 3 times the minimum dose approved as a treatment agent for pain, respectively.

### (Example 5: Evaluation of drug efficacy by FMR-1 knockout mice)

It is known that patients with human fragile X syndrome (FXS), which is caused by abnormalities of the X chromosome, present with social impairment symptoms, such as those often seen in ASD (Non-patent Documents 7 to 9). The Fmr1 knockout mouse (Cell, 1994, 15; 78 (1): 23-33) is a model mouse that mimics human fragile X syndrome (FXS), and is also widely used as a model of ASD. Buprenorphine hydrochloride, a MOR partial agonist used as an analgesic, and physiological saline of a solvent were administered once, and the action on social dysfunction of Fmr1 knockout mice was examined. The dose was calculated as a free form for buprenorphine. As a social function evaluation, a 3 chamber test (Nature Medicine, 2017, 23; p674-677) was used. In the 3chamber test, two types of mice (Unfamiliar and Familiar) are placed in cups at both left and right ends of three compartments, and a ratio of time to search for a mouse with high novelty to a mouse with low novelty is calculated and quantified as social function (preference to a mouse with high novelty).

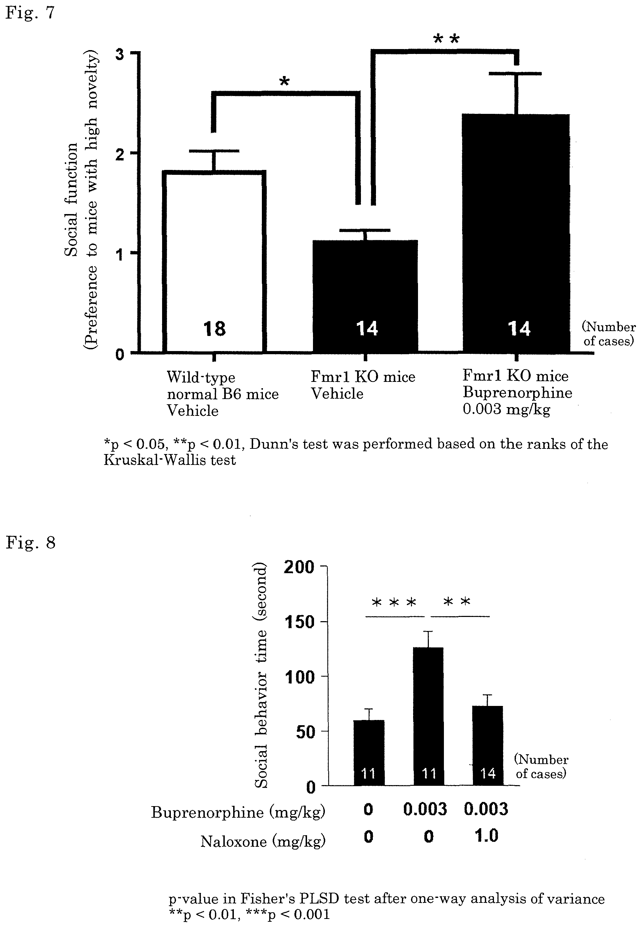

The results are shown in Fig. 7. The Fmr1 knockout mice exhibited social impairment as compared with the wild-type normal B6 mice, and a significant improvement effect in social impairment was observed by subcutaneous administration of buprenorphine. Since the present drug efficacy was observed at a low dose of less than 1/10 of the dose (1 mg/kg) at which the analgesic effect of buprenorphine was maximized, a characteristic action in a low dose range was observed.

### (Example 6: Antagonist test)

In the same manner as in Example 1, to the VPA model, Saline or a MOR selective antagonist, naloxone hydrochloride was subcutaneously administered so as to be 1 mg/kg as a hydrochloride, and 15 minutes after that, 0.003 mg/kg of buprenorphine was subcutaneously administered, and the amount of social behavior for 1 hour from 20 minutes after administration of buprenorphine was quantified.

The results are shown in Fig. 8. The improvement effect in social impairment by administration of 0.003 mg/kg of buprenorphine was antagonized by combined use of naloxone. From this, it was shown that buprenorphine exhibits an improvement effect in social impairment by MOR activation.

### (Example 7: Analysis of brain activation region)

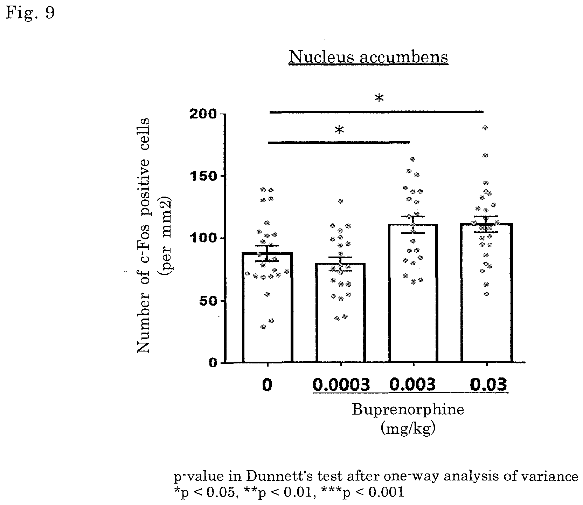

Saline or buprenorphine at 0.0003, 0.003, or 0.03 mg/kg was administered to the VPA model, and 1 hour after that, the brain was extracted and immunostained with an anti-c-Fos antibody, and the number of c-Fos positive cells in nucleus accumens (NAc) and medial prefrontal cortex, which are brain regions known to promote social behavior, and dorsomedial periaqueductal gray (dmPAG), which is a brain region known to reduce social behavior and to be involved in the expression of analgesic effects, was quantified as the number of activated nerve cells. The results are shown in Fig. 9 to 11.

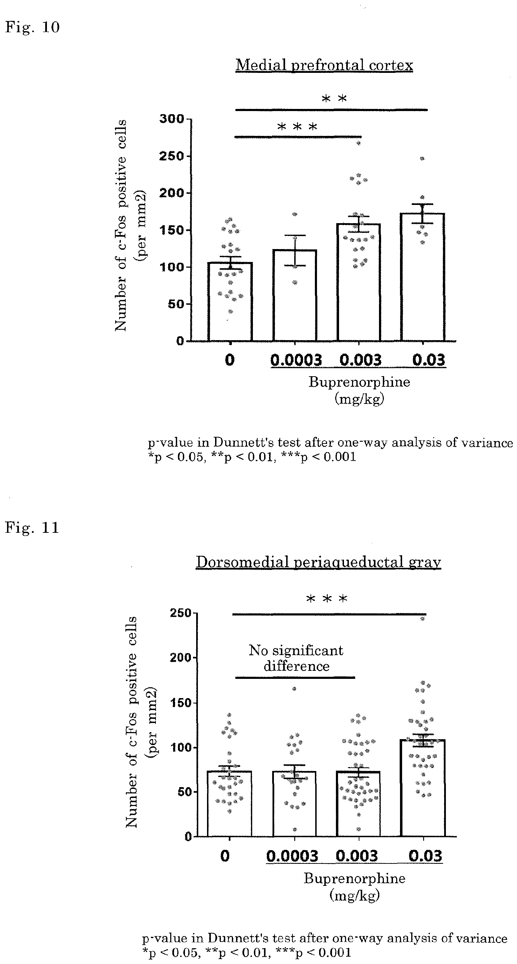

As shown in Figs. 9 and 10, in the nucleus accumbens and the medial prefrontal cortex, which are brain sites responsible for promoting social behavior in the previous studies (Coley AA, Padilla-Coreano N, Patel R, Tye KM. Valence processing in the PFC: Reconciling circuit-level and systems-level views. Int Rev Neurobiol. 2021; 158: 171-212. doi: 10.1016/bs.irn.2020.12.002. Epub 2021 Feb 4. PMID: 33785145), the number of activated nerve cells significantly increased at 0.003 mg/kg, which is a dose for improving social impairment. As shown in Fig. 10, in the dorsomedial periaqueductal gray, which is a brain site known to suppress social behavior in previous studies (Franklin TB, Silva BA, Perova Z, Marrone L, Masferrer ME, Zhan Y, Kaplan A, Greetham L, Verrechia V, Halman A, Pagella S, Vyssotski AL, Illarionova A, Grinevich V, Branco T, Gross CT. Prefrontal cortical control of a brainstem social behavior circuit. Nat Neurosci. 2017 Feb; 20 (2): 260-270. doi: 10.1038/nn.4470. Epub 2017 Jan 9. PMID: 28067904; PMCID: PMC5580810), there was no change in the number of activated nerve cells at 0.003 mg/kg.

On the other hand, at the time of administration of 0.03 mg/kg, which has an analgesic effect but has a reduced improvement effect in social impairment, the number of activated nerve cells significantly increased not only in the nucleus accumbens and medial prefrontal cortex but also in the dorsomedial periaqueductal gray.

From the above, in order to improve the social impairment using buprenorphine, it is considered that a dose that activates the nucleus accumbens and the medial prefrontal cortex but does not activate the dorsomedial periaqueductal gray is preferable. Since it is known that dorsomedial periaqueductal gray activation is important for the development of an analgesic effect by opioids in previous studies (de Freitas RL, Medeiros P, Khan AU, Coimbra NC. µ1-Opioid receptors in the dorsomedial and ventrolateral columns of the periaqueductal grey matter are critical for the enhancement of post-ictal antinociception. Synapse. 2016 Dec; 70 (12): 519-530. doi: 10.1002/syn.21926. Epub 2016 Sep 6. PMID: 27503688), a dose lower than the dose of buprenorphine used for an analgesic purpose is considered to be preferable for improvement in social impairment.

### (Example 8 Test to investigate the effect of low dose buprenorphine administration on symptoms of ASD)

To investigate the effect of ASD when low dose of buprenorphine is administered to a child with autism spectrum disorder, the following test is performed.

Dozens of children diagnosed with autism spectrum disorder by the DSM-5 criteria is targeted and the effect of buprenorphine administration is verified while performing clinical and biological monitoring. Buprenorphine is transdermally administered to a subject by a transdermal absorption type patch at a patch dose of 0.56 mg or 1.7 mg and a release rate of about 0.56 µg/h or about 1.7 µg/h. For example, the buprenorphine patch (Patch dose 5 mg, release rate 5 µg/h) may be administered in an amount of 1/9 or 1/3 of that amount. The presence or absence of symptom change due to dosing is examined using a standard symptom evaluation scale for ASD, for example, SRS-2(Social Responsiveness Scale Second Edition), CARS-2(Childhood Autism Rating Scale Second Edition), RBS-R (Repetitive Behavior Scale-Revised), or the like. With respect to the change in the line-of-sight pattern in an ASD patient for which the possibility as an objective symptom evaluation index is suggested, the presence or absence of a change due to dosing is examined using a line-of-sight measuring device.

### (Formulation Example)

The following Formulation Examples are merely examples and not intended to limit the scope of the invention.

Buprenorphine or the pharmaceutically acceptable salt thereof and/or morphine or the pharmaceutically acceptable salt thereof can be administered as a pharmaceutical composition by any conventional route, for example, orally, for example, in a form of a tablet or a capsule; parenterally, for example, in a form of an injectable preparation or a suspension; and topically, for example, in a form of a lotion, a gel, an ointment, or a cream, or in a transnasal form or a suppository form. The pharmaceutical composition comprising buprenorphine or the pharmaceutically acceptable salt thereof in free form or in the form of a pharmaceutically acceptable salt and/or morphine or pharmaceutically acceptable salt thereof in a free form or in a form of a pharmaceutically acceptable salt can be produced together with at least one pharmaceutically acceptable carrier or diluent in a conventional manner by a mixing, granulating, or coating method. For example, the oral composition can be a tablet, a granule, or a capsule, each comprising an excipient, a disintegrant, a binder, a lubricant, and the like, as well as an active ingredient and the like. Furthermore, the injectable composition can be a solution or a suspension, may be sterilized, and may contain a preservative, a stabilizer, a buffering agent, and the like.

### [INDUSTRIAL APPLICABILITY]

The pharmaceutical composition of the present invention comprising buprenorphine or the pharmaceutically acceptable salt thereof and/or morphine or the pharmaceutically acceptable salt thereof, and the method for administering buprenorphine or the pharmaceutically acceptable salt thereof and/or morphine or the pharmaceutically acceptable salt thereof are useful for the treatment and/or prevention of autism spectrum disorder, fragile X syndrome, and/or the autism spectrum disorder-like symptom. Furthermore, the pharmaceutical compositions or methods of the present invention are pharmaceutical preparations or treatment methods with low effective concentrations and high safety.

## Claims

1. A pharmaceutical composition for treatment and/or prevention of autism spectrum disorder, fragile X syndrome, and/or an autism spectrum disorder-like symptom, comprising buprenorphine or a pharmaceutically acceptable salt thereof and/or morphine or a pharmaceutically acceptable salt thereof.

2. The pharmaceutical composition according to claim 1, for the treatment and/or prevention of autism spectrum disorder.

3. The pharmaceutical composition according to claim 1 or 2, for administering to a patient diagnosed with autism spectrum disorder.

4. The pharmaceutical composition according to any of claims 1 to 3, for administering buprenorphine or the pharmaceutically acceptable salt thereof and/or morphine or the pharmaceutically acceptable salt thereof at a dose less than or equal to an effective amount for treating pain.

5. The pharmaceutical composition according to any of claims 1 to 4, for administering buprenorphine or the pharmaceutically acceptable salt thereof and/or morphine or the pharmaceutically acceptable salt thereof in a dosage amount less than or equal to about 1/2 times of an effective amount for treating pain.

6. The pharmaceutical composition according to any of claims 1 to 5, for administering buprenorphine or the pharmaceutically acceptable salt thereof and/or morphine or the pharmaceutically acceptable salt thereof in a dosage amount less than or equal to a minimum effective amount for treating pain.

7. The pharmaceutical composition according to any of claims 1 to 6, for administering buprenorphine or the pharmaceutically acceptable salt thereof and/or morphine or the pharmaceutically acceptable salt thereof in a dosage amount of about 1/30 to about 3 times of the minimum effective amount for treating pain.

8. The pharmaceutical composition according to any of claims 1 to 7, wherein buprenorphine or the pharmaceutically acceptable salt thereof and/or morphine or the pharmaceutically acceptable salt thereof is buprenorphine or the pharmaceutically acceptable salt thereof.

9. The pharmaceutical composition according to claim 8, wherein the pharmaceutical composition provides a maximum plasma concentration of buprenorphine or the pharmaceutically acceptable salt thereof of about 1 ng/mL or less.

10. The pharmaceutical composition according to claim 8 or 9, wherein the pharmaceutical composition provides a maximum plasma concentration of buprenorphine or the pharmaceutically acceptable salt thereof of about 4 pg/mL to about 0.6 ng/mL.

11. The pharmaceutical composition according to any of claims 8 to 10, wherein the pharmaceutical composition provides a maximum plasma concentration of buprenorphine or the pharmaceutically acceptable salt thereof of about 0.2 ng/mL or less.

12. The pharmaceutical composition according to any of claims 8 to 11, wherein the pharmaceutical composition provides a maximum plasma concentration of buprenorphine or the pharmaceutically acceptable salt thereof of about 8 pg/mL to about 0.2 ng/mL.

13. The pharmaceutical composition according to any of claims 8 to 12,
wherein the pharmaceutical composition is any of the following a) to c):
a) a pharmaceutical composition for transdermal administration comprising about 0.01 mg to about 10 mg of buprenorphine or the pharmaceutically acceptable salt thereof;
b) a pharmaceutical composition for transdermal administration that releases buprenorphine or the pharmaceutically acceptable salt thereof at about 0.01 µg/h to about 10 µg/h; and
c) a pharmaceutical composition for transmucosal administration comprising about 0.0001 mg to 0.1 mg of buprenorphine or the pharmaceutically acceptable salt thereof.

14. The pharmaceutical composition according to any of claims 8 to 12, wherein the pharmaceutical composition is either
a) a pharmaceutical composition for transdermal administration comprising about 0.56 mg to about 1.7 mg of buprenorphine or the pharmaceutically acceptable salt thereof, or
b) a pharmaceutical composition for transdermal administration that releases buprenorphine or the pharmaceutically acceptable salt thereof at about 0.56 µg/h to about 1.7 µg/h.

15. The pharmaceutical composition according to any of claims 8 to 12, wherein the pharmaceutical composition is either
a) a pharmaceutical composition for transdermal administration comprising about 0.56 mg or about 1.7 mg of buprenorphine or the pharmaceutically acceptable salt thereof, or
b) a pharmaceutical composition for transdermal administration that releases buprenorphine or the pharmaceutically acceptable salt thereof at about 0.56 µg/h or about 1.7 µg/h.

16. The pharmaceutical composition according to any of claims 8 to 15, for administration at an administration interval of 12 hours or more.

17. The pharmaceutical composition according to any of claims 8 to 16, wherein the drug efficacy lasts for 12 hours or more after administration.

18. The pharmaceutical composition according to any of claims 8 to 17, for administering buprenorphine or the pharmaceutically acceptable salt thereof without combination use with other drugs.

19. The pharmaceutical composition according to any of claims 1 to 7, wherein buprenorphine or the pharmaceutically acceptable salt thereof and/or morphine or the pharmaceutically acceptable salt thereof is morphine or the pharmaceutically acceptable salt thereof.

20. The pharmaceutical composition according to claim 19, wherein the pharmaceutical composition provides a maximum plasma concentration of morphine or pharmaceutically acceptable salt thereof of about 200 ng/mL or less.

21. The pharmaceutical composition according to claim 19 or 20, wherein the pharmaceutical composition provides a maximum plasma concentration of morphine or the pharmaceutically acceptable salt thereof of about 1 ng/mL to about 200 ng/mL.
